# EUROPEAN PATENT SPECIFICATION

(11) **EP 3 272 857 B1**
(45) Date of publication and mention of the grant of the patent: **12.08.2020**
(21) Application number: 16765008.4
(22) Date of filing: 16.03.2016
(51) Int. Cl.: C12N 5/0784, A61K 35/15, A61P 35/00, A61P 35/02, A61P 37/02, A61P 43/00, A61K 39/00

(54) **METHOD FOR PREPARING DENDRITIC CELL BY NON-ADHESIVE CULTURE USING IFN**
VERFAHREN ZUR HERSTELLUNG EINER DENDRITISCHEN ZELLE DURCH NICHTADHÄSIVE KULTUR UNTER VERWENDUNG VON IFN
MÉTHODE DE PRÉPARATION DE CELLULES DENDRITIQUES PAR CULTURE NON-ADHÉSIVE UTILISANT DE L'IFN

(30) Priority: 17.03.2015 JP 2015053804
(43) Date of publication of application: 24.01.2018
(73) Proprietor: Shimodaira, Shigetaka, Nagano-shi, Nagano 380-0928 (JP)
(72) Inventor: SHIMODAIRA, Shigetaka, Matsumoto-shi Nagano 390-8621 (JP); KOYA, Terutsugu, Matsumoto-shi Nagano 390-8621 (JP)
(74) Representative: J A Kemp LLP
(86) International application number: PCT/JP2016/058295
(87) International publication number: WO 2016/148179

(56) References cited:
- WO-A1-2014/126250
- WO-A2-2008/005859
- JP-A- H06 192 300
- JP-A- 2006 519 235
- JP-A- 2010 538 022
- TERUTSUGU KOYA ET AL: "Interferon-[alpha]-inducible Dendritic Cells Matured with OK-432 Exhibit TRAIL and Fas Ligand Pathway-mediated Killer Activity", SCIENTIFIC REPORTS, vol. 7, no. 1, 13 February 2017 (2017-02-13), XP055505710, DOI: 10.1038/srep42145
- CHANGHEE YOO ET AL: "Efficacy of Dendritic Cells Matured Early with OK-432 (Picibanil ), Prostaglandin E 2 , and Interferon-[alpha] as a Vaccine for a Hormone Refractory Prostate Cancer Cell Line", JOURNAL OF KOREAN MEDICAL SCIENCE, vol. 25, no. 9, 1 January 2010 (2010-01-01), page 1284, XP055505713, SEOUL, KR ISSN: 1011-8934, DOI: 10.3346/jkms.2010.25.9.1284
- AHAD M A ET AL: "Interferon to PEG-Interferon: A Review", TAJ: JOURNAL OF TEACHERS ASSOCIATION, RAJSHAHI MEDICAL COLLEGE, BD, vol. 17, no. 2, 1 December 2004 (2004-12-01), pages 113-116, XP002734715, ISSN: 1019-8555, DOI: 10.3329/TAJ.V17I2.3460
- Bukowski: "Treating Cancer with PEG Intron Pharmacokinetic Profile and Dosing Guidelines for an Improved", , 15 July 2002 (2002-07-15), XP055506404, Retrieved from the Internet: URL:https://onlinelibrary.wiley.com/doi/pd f/10.1002/cncr.10663 [retrieved on 2018-09-12]
- SAKAKIBARA MITSURU ET AL: "Quick generation of fully mature dendritic cells from monocytes with OK432, low-dose prostanoid, and interferon-alpha as potent immune enhancers", JOURNAL OF IMMUNOTHERAPY, LIPPINCOTT WILLIAMS & WILKINS, US, [Online] vol. 29, no. 1, 1 January 2006 (2006-01-01), pages 67-77, XP009507943, ISSN: 1524-9557, DOI: 10.1097/01.CJI.0000183093.77687.46 Retrieved from the Internet: URL:https://epo.summon.serialssolutions.co m/2.0.0/link/0/eLvHCXMwtZ1ta9swEMdFS6GMwdi 6wdZtcC-6vkkc5IdEzd6Ukj5SyrItMPbKOLbMvLTWa BqGP0S_c-8kSwrrSrdCIZggHGH7frnTybq_GNvqT3O RTcUg2ImKBBMUngXT_pAHPAtLUeSxEFpi4_somRyJL 0e0W4MV8_dtj2ppbENbU-Xsf1jbdYoN-B1tjke0Oh7 _ye6fF1U-a9Wk7WiQ8symc6Y1PNHD1IXe36Azkufnc xy6qgv6a6u>
- GAUZZI M. C. ET AL.: 'Suppressive Effect of 1alpha, 25-Dihydroxyvitamin D3 on Type I IFN- Mediated Monocyte Differentiation into Dendritic Cells: Impairment of Functional Activities and Chemotaxis' THE JOURNAL OF IMMUNOLOGY vol. 174, 2005, pages 270 - 276, XP055311993
- FARKAS A. ET AL.: 'Interferon-alpha in the generation of monocyte-derived dendritic cells: recent advances and implications for dermatology' BRITISH JOURNAL OF DERMATOLOGY vol. 165, 2011, pages 247 - 254, XP055312004
- HIROSHI SAITO ET AL.: 'Jujo Saibo no Seijuku to Kino, Jusho Zensoku to Churg-Strauss Syndrome Kanja-kan ni Mirareru Soi' JAPANESE JOURNAL OF ALLERGOLOGY vol. 53, 2004, page 893, XP055491755

## Description

### Technical Field

The present invention relates to a method for preparing dendritic cells from monocytes.

### Background Art

Dendritic cells (DCs) are potent antigen presenting cells *in vivo* and they are known to induce immune responses by presenting antigens to T cells. DCs are also known to directly react with, for example, B cells, NK cells, and NKT cells, in addition to T cells, and play a key role in immune responses. When immature DCs are stimulated by antigens, expression levels of CD40, CD80, CD86, and the like are elevated. DCs acquire a high degree of T cell stimulating capacity, migrate to peripheral lymph tissue, and activate T cells specific for the antigens incorporated therein. Thus, immune responses are induced.

In general, several types of cytokines are known as substances that are approved to be capable of inducing dendritic cell differentiation from blood progenitor cells. For example, many reports have been made concerning induction of DC differentiation with the use of GM-CSF in combination with IL-4 (Akagawa, K. S. et al., Blood, Vol. 88, No. 10, November 15, 1996: pp. 4029-4039). In addition, substances capable of inducing DC differentiation alone or in combination with other cytokines have been reported (O'Neill D. W. et al., Blood, Vol. 104, No. 8, October 15, 2004, pp. 2235-2246). For example, TNF-α, IL-2, IL-3, IL-6, IL-7, IL-12, IL-13, IL-15, HGF (hepatocyte growth factor), CD40 ligand, M-CSF, Flt3 ligand, c-kit ligand, and TGF-β have been reported.

DC differentiation is induced with the use of GM-CSF in combination with IL-4 via adhesive culture, mononuclear cells (monocytes and lymphocytes) are inoculated into a culture dish, lymphocytes are washed, and the adhered monocytes are used for culture. Culture is conducted in the presence of GM-CSF/IL-4 for 5 to 7 days, cells are washed with a medium and collected via scraping (physical peeling), the medium is exchanged with a fresh medium containing an adjuvant (an immunopotentiating agent, OK432), and mature DCs are thus prepared.

### Summary of the Invention

### Objects to Be Attained by the Invention

The present invention provides a method for preparing dendritic cells from monocytes via non-adhesive culture using interferon α.

### Means for Attaining the Objects

In the past, dendritic cells (DCs) were prepared from monocytes in the peripheral blood via adhesive culture with the use of GM-CSF and IL-4. Specifically, mononuclear cells (i.e., monocytes and lymphocytes) were inoculated into a culture dish, the lymphocytes and included platelets were washed, the adhered monocytes were cultured in the presence of GM-CSF and IL-4 for 5 days, the cells were washed with a medium and collected via scraping (physical peeling), the medium was exchanged with a fresh medium containing an adjuvant (an immunopotentiating agent) OK432, and mature DCs were thus prepared.

According to such technique, however, a DC yield was not sufficient. In order to use DCs for cancer therapy, in addition, DCs having activity such as cytotoxicity, in addition to a strong antigen presenting activity and phagocytic activity, have been desired.

The present inventors had conducted concentrated studies in order to enhance a DC yield and to prepare highly functional DCs. As a result, they discovered that optimized DCs could be prepared within a short period of time with the use of GM-CSF and pegylated interferon α (PEG-IFN-α) instead of GM-CSF and IL-4, monocytes are isolated and refined from the peripheral blood, and DCs are then prepared via non-adhesive culture; i.e., float culture. In addition, they discovered that an yield for DC preparation would be enhanced, and the resulting DCs would have a strong cytotoxic activity. This has led to the completion of the present invention.

Specifically, the present invention is as follows.
[1] A method for preparing cytotoxic dendritic cells from monocytes comprising subjecting monocytes which were isolated from the peripheral blood to non-adhesive culture in the presence of GM-CSF and pegylated interferon α and further conducting non-adhesive culture with the addition of prostaglandin E2 and OK432.
[2] The method for preparing dendritic cells from monocytes according to [1], wherein, following the non-adhesive culture conducted for 2 to 5 days in the presence of GM-CSF and pegylated interferon α, non-adhesive culture is conducted for an additional 1 or 2 days with the addition of prostaglandin E2 and OK432.
[3] The method for preparing dendritic cells from monocytes according to [1] or [2], wherein the monocytes are cultured with the use of 100 U/ml to 10,000 U/ml of GM-CSF, 500 ng/ml to 5 µg/ml of pegylated interferon α, 5 ng/ml to 50 ng/ml of prostaglandin E2, and 5 µg/ml to 50 µg/ml of OK432.
[4] The method for preparing dendritic cells from monocytes according to any one of [1] to [3], wherein the percentage of viable dendritic cells is 90% or higher and the yield indicating the proportion of the number of the obtained dendritic cells relative to the number of monocytes at the time of culture is 15% or higher.
[5] The method for preparing dendritic cells from monocytes according to any one of [1] to [4], wherein the obtained dendritic cells are positive for CD11c, CD14, CD40, CD56, CD80, CD86, HLA-ABC, and HLA-DR and negative for CD83 and CD197.
[6] The method for preparing dendritic cells from monocytes according to any one of [1] to [5], wherein, when effector cells (DCs) and target cells (the chronic myeloid leukemia cell line K562) are subjected to culture at a ratio of 50:1 for 18 hours, the cytotoxicity of the dendritic cells measured as the proportion of the target cells as dead cells is 30% or higher.
[7] An agent for inducing and differentiating cytotoxic dendritic cell (DC) from monocytes comprising GM-CSF, pegylated interferon α, prostaglandin E2, and OK432.
[8] The agent for inducing cytotoxic dendritic cell differentiation from monocytes according to [7], which comprises an agent for inducing and differentiating immature dendritic cell comprising GM-CSF and pegylated interferon α and an agent for dendritic cell maturation comprising prostaglandin E2 and OK432.

This description includes part or all of the disclosure of Japanese Patent Application No. 2015-053804, which is a priority document of the present application.

### Effects of the Invention

The method for preparing dendritic cells (DCs) of the present invention comprises subjecting the isolated and refined monocytes to non-adhesive culture in the presence of GM-CSF, pegylated interferon (IFN)-α (PEG-IFN-α), prostaglandin E2 (PGE2), and OK432. According to such method, DCs with high cytotoxicity can be prepared within a short period of time with a high yield. The obtained DCs can be preferably used for cancer immunotherapy.

### Brief Description of the Drawings

Fig. 1 schematically shows a method for preparing IL-4-OK DC and a method for preparing IFN-OK DC.
Fig. 2 shows the effects of an IFN-α formulation and OK432 used for dendritic cell preparation on cytotoxicity.
Fig. 3 shows the results of examination of the concentration of PEGINTRON used for dendritic cell preparation.
Fig. 4 shows cytotoxicity of the dendritic cells prepared via culture of monocytes for 1 to 6 days on K562 cells and MIA PaCa2.
Fig. 5 shows the effects of the PGE2 concentration when preparing dendritic cells.
Fig. 6 shows the phenotype of the prepared dendritic cells.
Fig. 7 shows the percentage of viable cells (Fig. 7A) and the yield (Fig. 7B) concerning the prepared dendritic cells.
Fig. 8 shows the antigen phagocytic activity (Fig. 8A) and the antigen degrading ability (Fig. 8B) concerning the prepared dendritic cells.
Fig. 9 shows *in vitro* CTL induction of the prepared dendritic cells.
Fig. 10 shows the capacity of the prepared dendritic cells for cytokine production.
Fig. 11 shows the cytotoxicity of the prepared dendritic cells that attacks cancer cells.
Fig. 12 shows the correlation between CD56 expression and cytotoxicity of the prepared dendritic cells. Fig. 12A shows CD56 expression (ΔMFI) of the dendritic cells prepared by various techniques, Fig. 12B shows the correlation between CD56 expression and cytotoxicity in IFN DC, and Fig. 12C shows the correlation between CD56 expression and cytotoxicity in IFN-OK DC.
Fig. 13 shows the cytotoxic mechanism of IFN-OK DC.
Fig. 14 shows a comparison between IL-4-OK DC and IFN-OK-DC.
Fig. 15-1 shows a procedure for preparing dendritic cells under various conditions.
Fig. 15-2 shows phase contrast microscopic images of the dendritic cells prepared under various conditions.
Fig. 16 shows the effects of OK432 on cytotoxicity.
Fig. 17 shows induction of cell damage caused by OK432.
Fig. 18 shows induction of cell damage caused by OK432 in the form of a box plot.
Fig. 19 shows the mean fluorescence intensity (MFI) of CD56 of DCs.
Fig. 20 shows the correlation between MFI of CD56 and cytotoxicity in mIFN-DC.
Fig. 21 shows the cluster formation by IFN-DC and K562 cells (the upper diagram) and the cluster formation by mIFN-DC and K562 cells (the lower diagram).
Fig. 22 shows K562 cell damage (Fig. 22A) and MIA PaCa-2 cell damage (Fig. 22B) caused by IFN-DC or mIFN-DC.

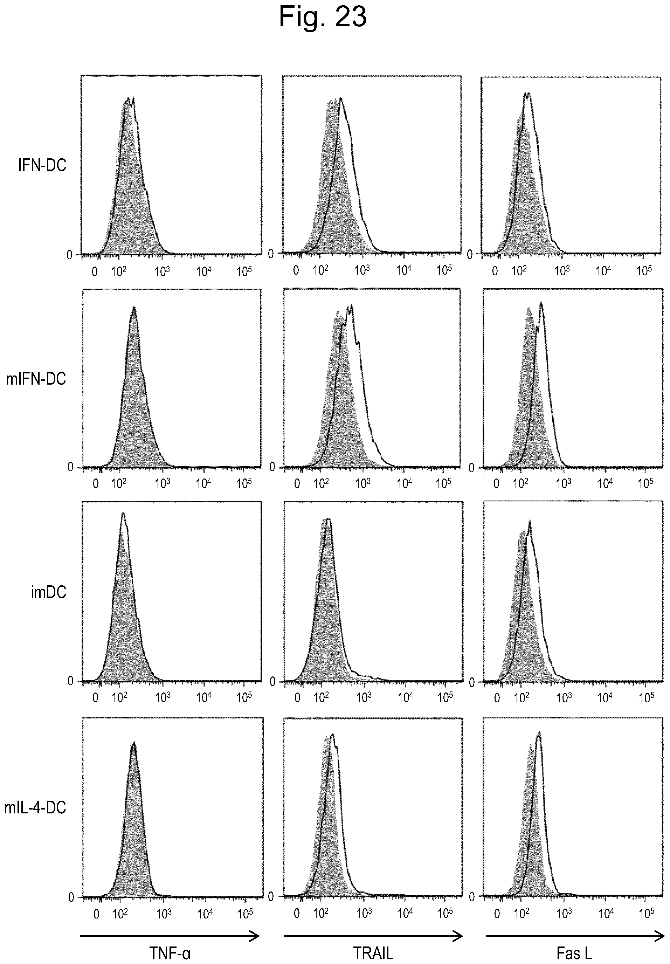

Fig. 23 shows TNF-α, TRAIL, and Fas ligand expression on the DC cell surface.
Fig. 24 shows DMFI of TRAIL (N = 9) (A) and Fas ligand (N = 8) (B) on the DC cell surface.
Fig. 25-1 shows the results of marker analysis of DCs prepared from PBMCs obtained from a cancer patient (CD11c (A), CD80 (B), and CCR7 (C)).
Fig. 25-2 shows the results of marker analysis of DCs prepared from PBMCs obtained from a cancer patient (CD14 (A), CD83 (B), and HLA-ABC (C)).
Fig. 25-3 shows the results of marker analysis of DCs prepared from PBMCs obtained from a cancer patient (CD40 (A), CD86 (B), and HLA-DR (C)).
Fig. 26-1 shows the antigen presenting activity of mIFN-DC and that of mIL-4-DC based on Mart-1-specific CTL induction.
Fig. 26-2 shows the antigen presenting activity of mIFN-DC and that of mIL-4-DC based on Mart-1-specific CTL induction (a dot plot).
Fig. 27 shows the antigen phagocytic activity and the antigen presenting activity of DCs.
Fig. 28 shows the cytokine-producing capacity of DCs.

### Embodiments for Carrying out the Invention

Hereafter, the present invention is described in detail.

The present invention concerns a method for preparing cytotoxic dendritic cells (DCs) from monocytes.

It has heretofore been known that dendritic cells were induced to differentiate from monocytes by subjecting monocytes to adhesive culture in the presence of the granulocyte macrophage colony-stimulating factors (GM-CSF) and interleukin 4 (IL-4). In the present invention, monocytes are subjected to non-adhesive culture with the use of GM-CSF and pegylated interferon (IFN)-α (PEG-IFN-α) for induction of dendritic cell differentiation. By conducting culture in the presence of GM-CSF and PEG-IFN-α, immature dendritic cells are prepared. Such immature dendritic cells are further subjected to non-adhesive culture in the presence of GM-CSF, PEG-IFN-α, prostaglandin E2 (PGE2), and OK432, so that mature DCs having cytotoxicity can be obtained.

PEG-IFN-α is composed of polyethylene glycol (PEG) bound to IFN-α. A preferable PEG-IFN-α is PEG-IFN-α-2b. As PEG-IFN-α, a commercially available PEG-IFN formulation can be used. An example of a commercially available PEG-IFN-α formulation is a PEG-IFN-α-2b formulation (i.e., PEG-Intron®; generic name: PEG interferon α-2b (genetic recombination)).

PEG-Intron® is represented by a structural formula: H₃C-(O-CH₂CH₂)n-OCO-Interferon alfa-2b, which is composed of a molecule of methoxy polyethylene glycol (average molecular weight: about 12,000) covalently bound to a single site of amino acid residues (Cys1, His7, Lys31, His34, Lys49, Lys83, Lys112, Lys121, Tyr129, Lys131, Lys133, Lys134, Ser163, and Lys164) of interferon α-2b (genetic recombination) (molecular weight: 19268.91) through a carbonyl group. The molecular weight thereof is about 32,000, and the molecular formula thereof is C₈₆0H₁₃₅₃N₂₂₉O₂₅₅S₉. The CAS registry number is 215647-85-1.

OK432 is a lyophilized powder of *Streptococcus pyogenes* (A group, type 3) Su strain cells treated with penicillin, which is a lyophilized cell formulation prepared by treating hemolytic streptococcus *Streptococcus pyogenes* (A group, type 3) Su strain cells in the presence of benzylpenicillin potassium under certain conditions. OK432 is used as an adjuvant. The CAS registry number is 39325-01-4. OK-432 is a clinical trial number, and commercially available Picibanil® can be used.

In the method of the present invention, monocytes that were purified and refined in advance are used. Examples of monocytes include peripheral blood-derived monocytes, bone marrow-derived monocytes, spleen cell-derived monocytes, and umbilical cord blood-derived monocytes, with the peripheral blood-derived monocytes being particularly preferable. Monocytes are positive for CD14. When monocytes are to be collected from organisms, accordingly, monocytes can be isolated on the basis of the presence of CD14 as the indicator with the use of a fluorescent activated cell sorter (FACS), a flow cytometer, a magnetic separator, or the like, and the isolated can then be refined. Also, monocytes can be concentrated and refined with the use of an apparatus for apheresis (blood component collection). In addition, monocytes can be isolated and refined via density gradient centrifugation with the use of Ficoll® or the like. Animal species from which monocytes are derived are not limited. For example, mammalian animals, such as mice, rats, guinea pigs, hamsters, rabbits, cats, dogs, sheep, pigs, cows, horses, goats, monkeys, and humans, can be used. As FACS and a flow cytometer, for example, FACS vantage (Becton, Dickinson and Company) and FACS Calibur (Becton, Dickinson and Company) can be used. An example of a magnetic separator that can be used is autoMACS® (Miltenyi Biotec). On the basis of CD14 expression as the indicator, for example, monocytes can be isolated from peripheral mononuclear cells (PBMCs) with the use of CD14 microbeads to which CD14 has been bound via techniques involving the use of autoMACS® and CliniMACS®.

The isolated and refined monocytes are subjected to non-adhesive culture; that is, float culture. Non-adhesive culture may be conducted with the use of a culture vessel, such as a non-adhesive plate, dish, or flask. A non-adhesive culture vessel is prepared by coating a culture dish surface with a compound, such as a superhydrophilic polymer, phospholipid polymer, or MPC polymer. Alternatively, a culture dish surface is made hydrophilic without the use of a coating agent, so as to prevent cells from adhering to the culture vessel. For example, a low-adhesive culture dish, such as HydroCell™ (CellSeed), EZ-BindShut® II (Iwaki), Nunclon™ Vita (NOF Corporation), or Lipidure®-Coat (NOF Corporation), can be used.

Examples of culture media that can be used include RPMI1640, DMEM, AIM-V®, and X-VIVO5®, and adequate antibiotics, animal blood sera, human albumin, human blood sera, or the like may be added to such basal media, and culture may be conducted therein.

When monocytes are used at the concentration of 10⁴ to 10⁷ cells/ml, for example, the concentration of GM-CSF used for culture is 100 U/ml to 10,000 U/ml, preferably 500 U/ml to 2,000 U/ml, more preferably 800 to 1,200 U/ml, and particularly preferably 1,000 U/ml. Alternatively, such concentration is 10 ng/ml to 1,000 ng/ml, preferably 20 ng/ml to 200 ng/ml, and more preferably 20 ng/ml to 100 ng/ml. The PEG-IFN-α concentration is 100 ng/ml to 10 µg/ml, preferably 500 ng/ml to 5 µg/ml, and more preferably 500 ng/ml to 2 µg/ml. The PG2E concentration is 1 ng/ml to 100 ng/ml, preferably 5 ng/ml to 50 ng/ml, and more preferably 5 ng/ml to 20 ng/ml. The OK432 concentration is 1 µg/ml to 100 µg/ml, preferably 5 µg/ml to 50 µg/ml, and more preferably 5 µg/ml to 20 µg/ml.

Culture is conducted in the presence of GM-CSF and PEG-IFN-α for 2 to 5 days, preferably for 3 to 4 days, and more preferably for 3 days. By conducting culture in the presence of GM-CSF and PEG-IFN-α, immature DCs are obtained. Such immature DCs are subjected to non-adhesive culture in the presence of GM-CSF, PEG-IFN-α, prostaglandin E2, and OK432 for an additional 1 or 2 days, and preferably 1 day. Thus, cytotoxic DCs can be obtained. OK-432 and PGE2 may be added by supplementing the medium containing GM-CSF and PEG-IFN-α with a supplemental medium containing OK-432 and PGE2. A total culture period is for 3 to 7 days, preferably 4 to 6 days, more preferably 4 to 5 days, and particularly preferably 4 days.

Expression of monocyte or DC surface antigens may be inspected via FACS or the like, so that the concentration at which cells with the degree of differentiation of interest are obtained can adequately be determined.

By the method described above, the isolated and refined monocytes are cultured in the presence of GM-CSF and PEG-IFN-α and further cultured in the presence of GM-CSF, PEG-IFN-α, PGE2, and OK432. Thus, cytotoxic DCs are differentiated and induced. The DCs obtained in such a manner are occasionally referred to as "IFN-OK-DCs" or "mIFN-DCs." The DCs prepared in such a manner of the present invention are characterized in that they have dendrites in morphological sense. By analyzing the DCs via FACS or other means, in addition, such DCs are found to be positive for CD11c, CD14, CD40, CD56, CD80, CD86, HLA-ABC, and HLA-DR and negative for CD83 and CD197 as surface antigens. In particular, CD56 which is an indicator of NK cell and CD86 and HLA-DR which are the minimal standards of DCs are expressed.

Whether or not the cells are positive or negative for such surface antigens can be determined via microscopic observation as to whether or not the cells are stained with the antibodies reacting with such antigens, which have been labeled with a color enzyme, a fluorescent compound, or the like. For example, cells may be subjected to immunostaining with the use of such antibodies, so as to determine the presence or absence of surface antigens. Further, magnetic beads comprising the antibodies bound thereto may be used. Furthermore, the presence or absence of surface antigens can be determined with the use of FACS or a flow cytometer. The expression "negative for surface antigens" means that the cells are not sorted as positive cells when the cells are analyzed with the use of FACS as described above, and expression is not observed when expression is analyzed via immunostaining. When the expression level is lower than the lower detection limit of such technique, the cells are determined to be negative for surface antigens.

The cytotoxicity of the DCs prepared by the method of the present invention is correlated with the expression level of CD56 as an NK cell marker. The cytotoxicity of the DCs prepared by the method of the present invention is 30% or higher, preferably 32% or higher, and more preferably 35% or higher, when measured as the proportion of the target cells as dead cells after the effector cells (DCs) and the target cells (i.e., the chronic myeloid leukemia cell line K562) are cultured at the proportion of 50:1 for 18 hours in accordance with the description of M. Korthals et al. Journal of Translational Medicine 2007, 5: 46.

The DCs prepared by the method of the present invention exert cytotoxicity with the aid of the tumor necrosis factor (TNF)-related apoptosis-inducing ligand (TRAIL) or a FAS ligand (FasL). TRAIL and FasL are referred to as "cytotoxic factors," and they are reported to function in certain types of DCs or lymphocytes when attacking cancer cells. When the DCs prepared by the method of the present invention exert cytotoxicity, specifically, TRAIL or a Fas ligand also reacts. Accordingly, the DCs prepared by the method of the present invention can effectively exert cytotoxicity on cancer cells expressing TRAIL receptors or FAS, although the targets are not limited thereto.

In the method of the present invention, in addition, DCs are prepared from monocytes via non-adhesive culture. Accordingly, the percentage of viable cells and the yield of DCs are also high. The percentage of viable cells of the obtained DCs is 70% or higher, preferably 80% or higher, more preferably 90% or higher, further preferably 95% or higher, and still further preferably 97% or higher, which is the standard designated by the National Institutes of Health (NIH). The DC recovery rate (i.e., the proportion of the number of the viable DCs obtained relative to the number of the monocytes inoculated) is 15% or higher, and preferably 20% or higher.

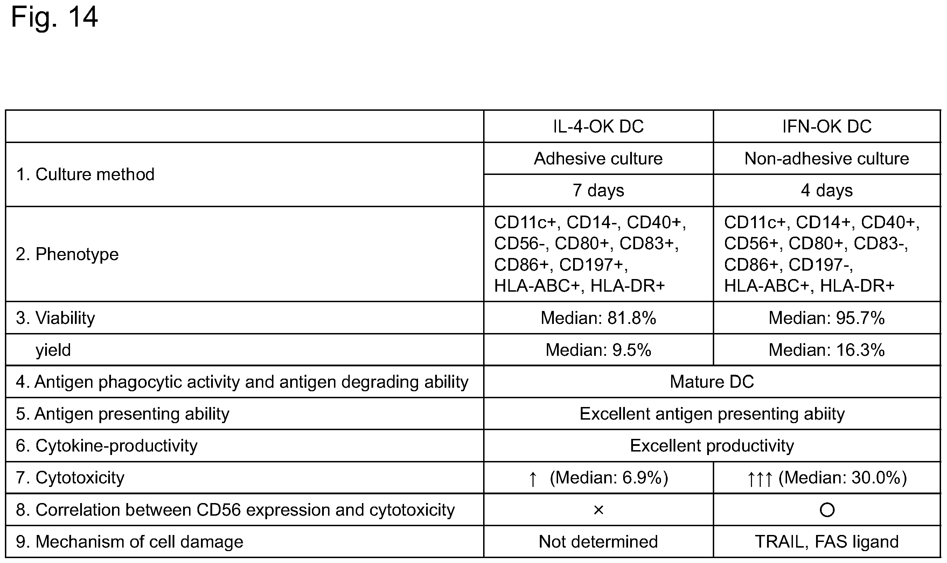

For example, there are characteristic differences as shown in Fig. 14 between DCs prepared via adhesive culture with the use of GM-CSF, IL-4, PGE2, and OK432 (IL-4-OK DC) and DCs prepared via non-adhesive culture with the use of GM-CSF, PEG-IFN, PGE2, and OK432 (IFN-OK DC).

While preparation of IL-4-OK DCs requires about 7 days, specifically, the IFN-OK DCs of the disclosure can be prepared within a short period of time (about 4 days). While the percentage of viable IL-4-OK DCs is about 82%, that of the IFN-OK DCs as disclosed herein is as high as about 95% or higher. While the yield of IL-4-OK DC preparation is about 10% (10% to 15%), in addition, that of IFN-OK DC preparation is high; that is, about 16% or higher (e.g., 10% to 25% or higher). While the cytotoxicity of IL-4-OK DCs is 10% or lower, further, that of IFN-OK DC is as high as about 30% or higher.

Regarding the antigens expressed on the cell surface, IL-4-OK DC is CD11c-positive, CD14-negative, CD40-positive, CD56-negative, CD80-positive, CD83-positive, CD86-positive, CD197-positive, HLA-ABC-positive, and HLA-DR-positive. In contrast, IFN-OK DC is CD11c-positive, CD14-positive, CD40-positive, CD56-positive, CD80-positive, CD83-negative, CD86-positive, CD197-negative, HLA-ABC-positive, and HLA-DR-positive. The cytotoxic level of IFN-OK DC is correlated with the CD56 expression level. In addition, IFN-OK DCs are found to exert cytotoxicity through TRAIL or a FAS ligand. This indicates that IFN-OK DCs exert the cytotoxicity on cancer cells expressing TRAIL or a FAS ligand.

In addition, the DCs as disclosed herein have antigen phagocytic activity and degrading activity equivalent to those of the DCs prepared with the use of mGM-CSF and IL-4 and excellent antigen presenting activity as with the DCs prepared with the use of mGM-CSF and IL-4.

The DCs as disclosed herein have the cytokine-producing ability and killer activity superior to those of the DCs prepared with the use of mGM-CSF and IL-4.

The present invention also concerns an agent for inducing and differentiating DC from monocytes comprising GM-CSF and PEG-IFN-α. Such agent for inducing and differentiating DC can also be referred to as an "agent for DC preparation." The agent for inducing and differentiating DC may further comprise PGE2 and OK432. The agent for inducing and differentiating DC may be composed of a first reagent comprising GM-CSF and PEG-IFN-α and a second reagent comprising PGE2 and OK432. Also disclosed herein is a kit for inducing and differentiating DC comprising the first reagent and the second reagent. The first reagent comprising GM-CSF and PEG-IFN-α is used for inducing and differentiating immature DCs and the second reagent comprising PGE2 and OK432 is used for maturation of immature DCs.

By the method of the present invention, DCs are induced as mature DCs. In addition, disclosed herein are DCs prepared by the method of the present invention and a cell population comprising such DCs. Such cell population includes 10% or more, 30% or more, 50% or more, 70% or more, 90% or more, or 95% or more DCs.

The DCs prepared by the method of the present invention can be used for dendritic cell therapy. An example of dendritic cell therapy is cancer immunotherapy that is known as dendritic cell vaccine therapy. For example, dendritic cells are prepared from monocytes obtained from a subject by the method of the present invention, and the prepared dendritic cells are returned into the subject' body. Thus, dendritic cells can be used for treatment or prevention of cancer. In such a case, the prepared dendritic cells can act regardless of cancer type and can exert cancer therapeutic effects. When preparing dendritic cells, alternatively, culture may be conducted with the addition of cancer-specific antigens that are specific to a particular type of cancer. Thus, dendritic cells exhibiting cancer-type-specific anticancer immunoreactivity can be prepared. Also, such cells can be used for treatment of bacterial or viral infections. For the treatment of infections, DCs prepared from monocytes via non-adhesive culture in the presence of GM-CSF, PEG-IFN-α, PGE2, and OK432 by the method of the present invention are useful. The prepared dendritic cells may be administered to a subject via the intradermal, subcutaneous, intravenous, intra-nodal, or other route. The amount and the time of administration can be adequately determined in accordance with the disease type, the disease severity, and the conditions of the subject.

### Examples

The present invention is described in greater detail with reference to the following examples, although the technical scope of the present invention is not limited to these examples.

### Example 1: Preparation of dendritic cells using IFN (Part 1)

### 1. Preparation of dendritic cells

After informed consent was obtained, the peripheral blood mononuclear cells (PBMCs) were obtained from cancer patients via blood component collection (apheresis), and dendritic cells (DCs) were prepared using the obtained PBMCs as raw materials (Medical ethics approval number: 2107; date of approval: September 4, 2012). With the use of IL-4 DCs obtained via culture of monocytes in the presence of the granulocyte macrophage colony-stimulating factor (GM-CSF) and interleukin-4 (IL-4), OK432 (Picibanil®) prepared via treatment of a spontaneous mutant (Su strain) of group A *Streptococcus pyogenes* with penicillin, and prostaglandin E2 (PGE2), mature IL-4-OK DCs were prepared via a conventional adhesive culture technique. PBMCs were suspended in the AIM-V medium (Invitrogen Life Technologies) at the monocyte density of 2 to 4 × 10⁶ cells/ml, the cell suspension was inoculated into an adhesive cell culture dish (BD Primaria™, BD Bioscience), non-adherent cells were removed 30 minutes later, the medium was exchanged with the AIM-V medium supplemented with GM-CSF (50 ng/ml; Gentaur) and IL-4 (50 ng/ml; R&D Systems) on the following day, the adhered monocytes were cultured for 5 days, and immature IL-4 DCs were then recovered with the use of a scraper. For maturation, the cells were suspended in a fresh AIM-V medium containing 10 µg/ml of OK432 (streptococcal preparation, Chugai Pharmaceutical Co, Ltd, Tokyo, Japan) and 50 ng/ml of PGE2 (Daiichi Fine Chemical Co, Ltd, Toyama, Japan), and culture was conducted for an additional 16 to 24 hours to prepare dendritic cells. The dendritic cells prepared in such a manner are referred to as "IL-4-OK DCs."

IFN DCs obtained via culture of monocytes in the presence of interferon (IFN)-α (IFN-α) and GM-CSF and IFN-OK DCs matured with the aid of OK432 and PGE2 were prepared with the use of the monocytes refined from PBMCs with the aid of CD14 microbeads (Miltenyi Biotec). With the use of the monocytes refined at the purity of 95% or higher on a daily basis, the cells were suspended in the AIM-V medium containing GM-CSF (1,000 U/ml; Miltenyi Biotec) and a pegylated interferon α-2b formulation Pegintron® (1 µg/ml; MSD) at 2 to 4 × 10⁶ cells/ml, the cell suspension was inoculated into a non-adhesive culture dish (HydroCell, CellSeed), and culture was conducted for 3 days to prepare IFN DCs. In addition, the IFN DC culture solution was supplemented with the maturation medium 4 days after the initiation of culture (after the culture had been conducted for 3 days), and culture was conducted for an additional 16 to 24 hours in the culture medium containing OK432 (final concentration: 10 µg/ml) and PGE2 (10 ng/ml) to prepare IFN-OK DCs. The dendritic cells prepared in such a manner are referred to as "IFN-OK DCs." In some experiments, dendritic cells were prepared in the same manner except for the use of an interferon α-2b formulation (Intron® A, 1,000 U/ml; MSD).

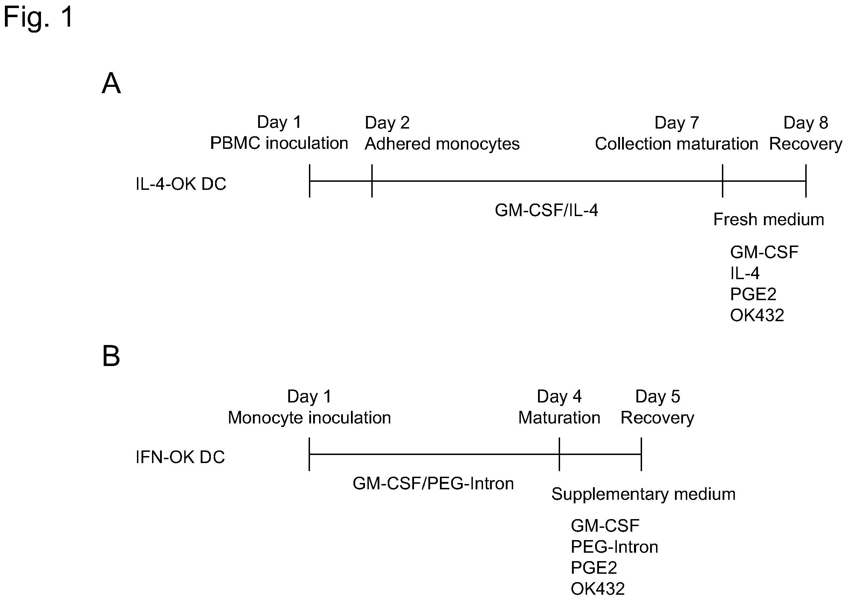

Fig. 1 schematically shows a method for IL-4-OK DC preparation (Fig. 1A) and a method for IFN-OK DC preparation (Fig. 1B).

In this example, the dendritic cells prepared with the use of IL-4 alone instead of IL-4 and OK432 and the dendritic cells prepared with the use of IFN alone instead of IFN and OK432 were examined, in addition to IL-4-OK DCs and IFN-OK DC. Such dendritic cells are referred to as IL-4 DC and IFN DC, respectively.

The type of IFN-α used, the concentration of INF-α added, the culture period, and the PGE2 concentration were examined.

Cytotoxicity of the prepared dendritic cells was examined in the manner described below.

### Cytotoxicity test

The chronic myeloid leukemia strain K562 (ATCC) or the human pancreatic cancer-derived cell strain MIA PaCa2 (RIKEN BRC) was suspended in PBS containing 0.1% FCS supplemented with carboxyfluorescein succinimidyl ester (CFSE) (5 µm; Molecular Probes), and the cell suspension was subjected to the reaction at 37°C for 10 minutes, followed by washing in the AIM-V medium. With the use of the AIM medium containing 10% FCS, 5 × 10⁵ DCs (effector cells) were mixed with CFSE-stained cancer cells (target cells) at a ratio (effector cells:target cells (E:T)) of 50:1. The cells were subjected to the reaction at 37°C for 18 hours, washed in a FACSFlow solution, and then stained with 2 µg/ml of propidium iodide (PI), so as to detect dead cells via FACS. The number of CFSE-positive and PI-positive cells was divided by the number of CFSE-positive cells, the resulting value was evaluated as the proportion of cytotoxicity. As a negative control, unstained cancer cells were designated as effector cells, the effector cells were mixed with the CFSE-stained cancer cells at a ratio E:T of 50:1, and the cells were subjected to the reaction in the same manner as described above.

The inhibition test using antibodies was carried out by subjecting 10 µg/ml anti-TRAIL antibodies or anti-FAS ligand antibodies (eBioscience) and DCs to incubation for 1 hour in advance, mixing the CFSE-labeled cancer cells therewith, and then conducting the reaction.

### (1) Selection of IFN-α formulation

IFN DCs were prepared with the use of two types of IFN-α formulations (i.e., GM-CSF and PEGINTRON (1 µg/ml) or Intron A (1,000 U/ml)), and cytotoxicity was examined with or without the addition of OK432. The CFSE-labeled chronic myeloid leukemia strain K562 was designated as the target, DCs were mixed with the target at various proportions, and the percentage of dead cells was determined 18 hours later to determine the cytotoxicity (%). An error bar represents "mean ± standard error," and experiments were carried out 3 times independently (representative data (n = 3), the significant difference was tested by the Tukey-Kramer test, * p < 0.01, ** p < 0.001).

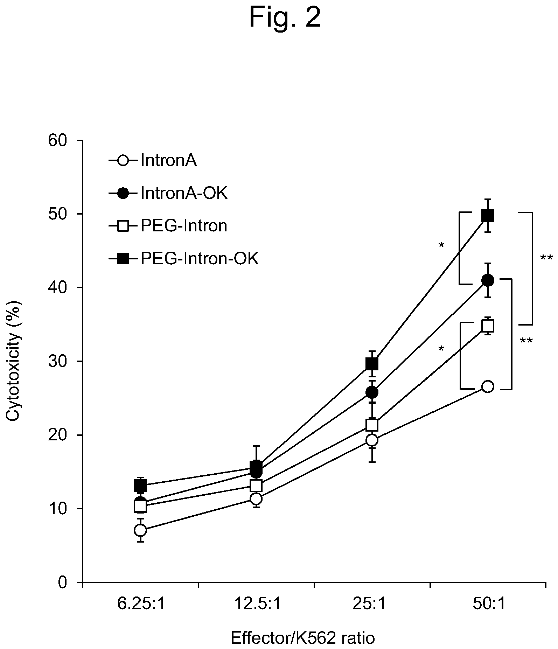

The results are shown in Fig. 2. As shown in Fig. 2, OK432 elevates cytotoxicity, and IFN DC using PEGINTRON exhibits higher cytotoxicity than Intron A. Based on such results, PEGINTRON was used as the IFN-α formulation.

### (2) Examination of PEGINTRON concentration

With the use of monocytes obtained from a healthy volunteer (Donor ^{#}4) and those obtained from a cancer patient (Patient ^{#}12), the optimal concentration of PEGINTRON was examined based on the cytotoxicity. Culture was conducted for 3 days at different concentrations, maturation with the aid of OK432 was conducted for 1 day, and IFN-OK DCs were then collected 4 days after the initiation of culture.

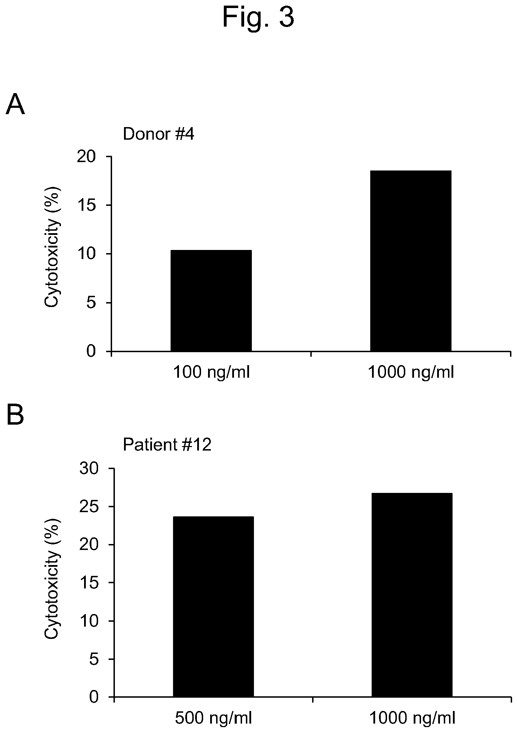

The results are shown in Fig. 3. Fig. 3A shows the results concerning Donor ^{#}4 and Fig. 3B shows the results concerning Patient ^{#}12. As shown in Fig. 3, the concentration (1,000 ng/ml (1 µg/ml)) at which a high degree of cytotoxicity was observed in the healthy volunteer and in the cancer patient was employed. At a concentration higher than the aforementioned level, cells died, and measurement could not be performed.

### (3) Examination of culture period

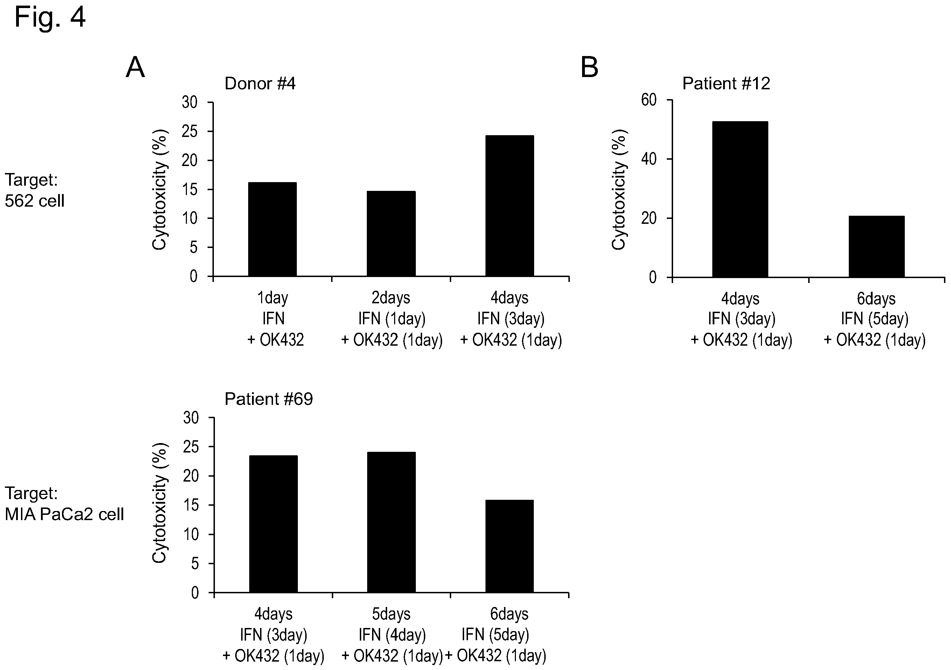

Monocytes were cultured for 1 to 6 days with the use of PEGINTRON and OK432, the cytotoxicity relative to the K562 cells as the target and the cytotoxicity relative to MIA PaCa2 as the target were determined. The results are shown in Fig. 4. The upper charts shown in Fig. 4 demonstrate the results attained with the use of the K562 cells as the target cells, (A) demonstrates the results concerning Donor ^{#}4, and (B) demonstrates the results concerning Patient ^{#}12. The lower chart shown in Fig. 4 demonstrates the results concerning Patient ^{#}69 attained with the use of MIA PaCa2 as the target cell.

When the K562 cells were used as the target cells, a high degree of cell damage was observed after culture had been conducted for 4 days (culture with IFN for 3 days, followed by culture with OK432 for 1 day) in both the healthy volunteer and the cancer patient.

A high degree of cytotoxicity was observed 4 days and 5 days after the initiation of culture on MIA PaCa2. From the viewpoint of a shorter culture period, a culture period of 4 days (culture with IFN for 3 days, followed by culture with OK432 for 1 day) was employed. The cytotoxicity on K562 and MIA PaCa2 was also confirmed.

### (4) Examination of PGE2 concentration

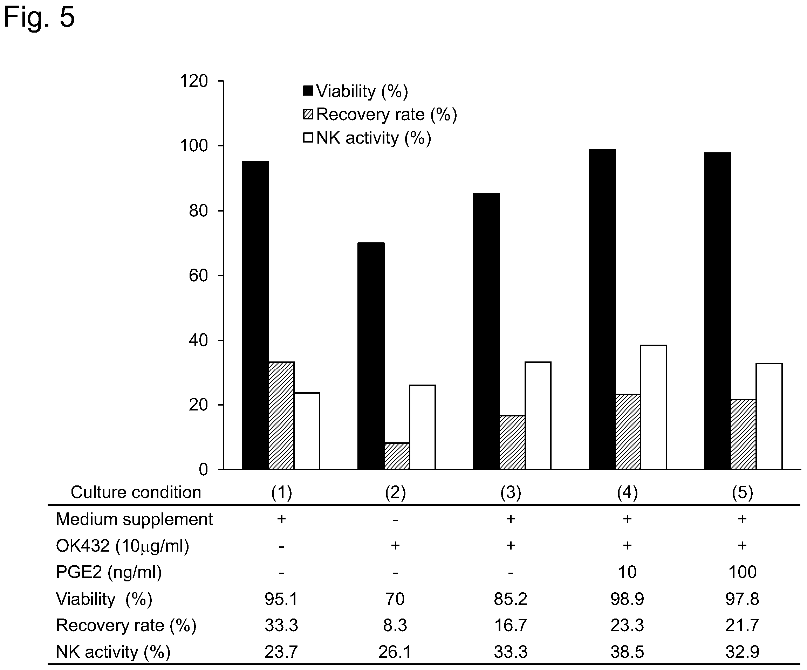

The influence of PGE2 imposed on the percentage of viable cells, the recovery rate, and the cytotoxicity was inspected. The results are shown in Fig. 5. A process of culture comprising adding the maturation medium on Day 4 of (4) IFN DC culture in the presence of 10 µg/ml (final concentration) of OK432 and 10 ng/ml of PGE2, which would provide the best results, was employed.

### 2. Analysis of DC phenotype

The prepared DCs were treated with the FcR Blocking Reagent (Miltenyi Biotec) for 10 minutes, and the cells were stained with the antibodies serving as indicators for the dendritic cells labeled with FITC (fluorescein isothiocyanate) or PE (phycoerythrin) (i.e., CD11c, CD80, CD86, and HLA-ABC (BD Pharmingen), CD14, CD40, CD83, and HLA-DR (eBioscience), and CD197 (R&D Systems)). As the indicator for the NK cell phenotype, APC-conjugated CD56 (BD Pharmingen) was used. Immediately before FACS analysis, dead cells were stained with 2 µg/ml of propidium iodide (PI, Sigma-Aldrich), and the cells were then analyzed using FACS CantoII (BD Bioscience).

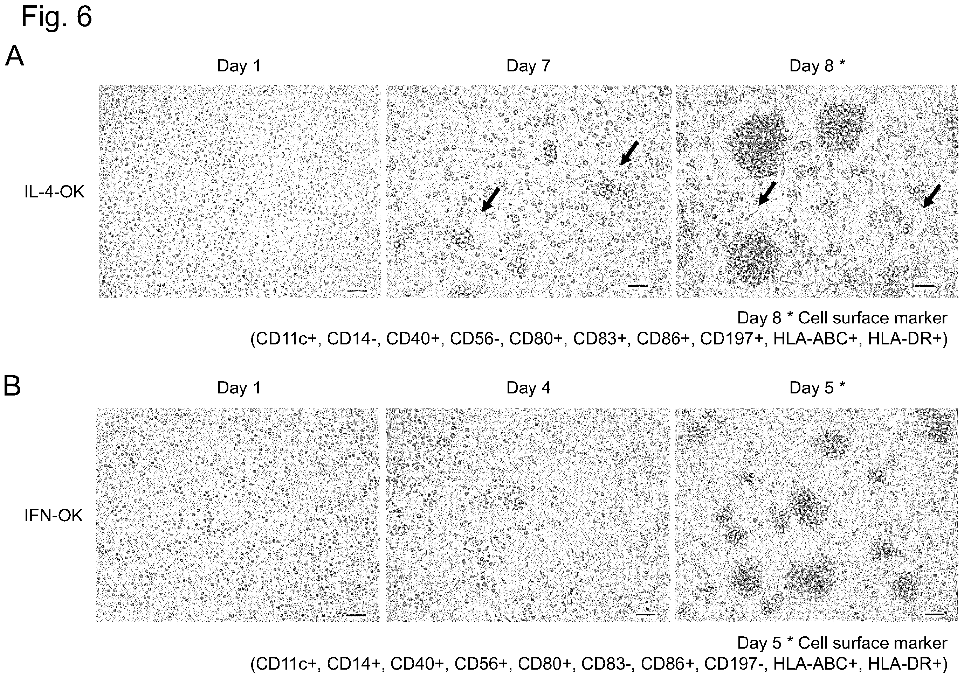

The results are shown in Fig. 6. Fig. 6A demonstrates the results concerning IL-4-OK DC, and, from left to right, the results attained on Day 1, those on Day 7, and those on Day 8. Fig. 6B demonstrates the results concerning IFN-OK DC C, and, from left to right, the results attained on Day 1, those on Day 4, and those on Day 5. After the addition of an adjuvant (an immunopotentiating agent) OK432, apparent cluster formation (cell aggregation) was observed in IL-4-OK DC on Day 8 and in IFN-OK DC on Day 5. A black arrow shown in Fig. 6A indicates cell adhesion. The application of physical damage at the time of recovery with a scraper was considered to lower the percentage of viable cells and the yield. In contrast, IFN-OK DCs prepared via non-adhesive culture did not adhere to a non-adhesive HydroCell dish throughout the culture period. Cell surface marker expression was inspected via FACS analysis with the use of the recovered DCs. As a result, expression of CD86 and HLA-DR as dendritic cell markers was observed, and differences were observed in expression of CD14, CD56, CD83, and CD197. Expression of CD56 serving as the NK cell marker known as a killer cell was observed in IFN-OK DC. A black scale bar represents 50 µm.

### 3. Percentage of viable cells and yield concerning the prepared dendritic cells

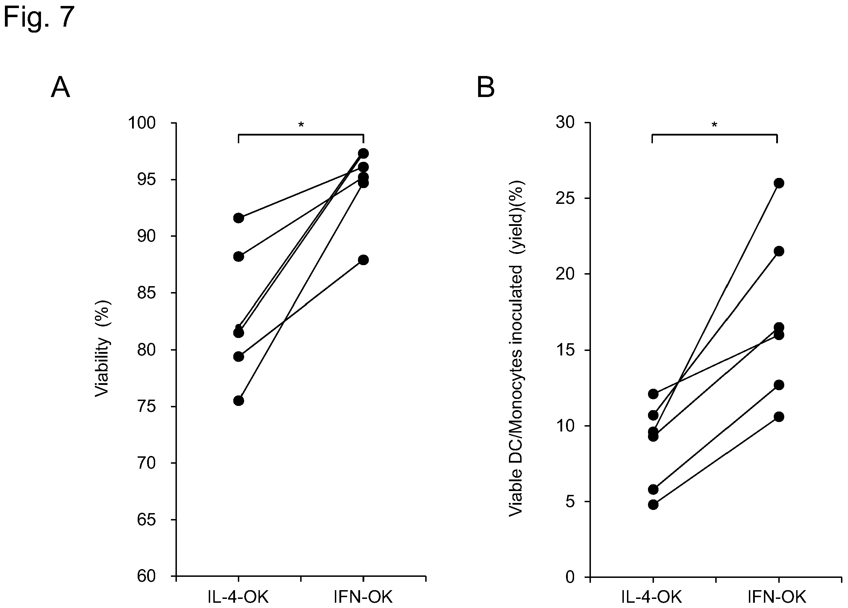

Fig. 7 shows the results of inspection of the percentage of viable cells concerning the prepared DCs via trypan blue staining. The percentage of viable IFN-OK DCs was found to be significantly higher than that of IL-4-OK DCs (Fig. 7A). On the basis of the percentage of viable DCs obtained vial culture relative to the number of inoculated monocytes, the yield (viable DCs/inoculated monocytes) was calculated. As a result, the percentage of viable IFN-OK DCs was found to be significantly higher than that of IL-4-OK DCs (Fig. 7B). In this example, DCs were prepared from monocytes obtained from cancer patients and analyzed (n = 6). Significant difference was tested by the Wilcoxon signed rank test. The symbol "*" indicates P < 0.05.

### 4. Evaluation of antigen phagocytic activity and antigen degrading ability

In order to inspect the antigen phagocytic activity, FITC-labeled dextran (M.W. = 40,000, Molecular Probes) was adjusted to 250 µg/ml using the AIM-V medium containing 10% fetal calf serum (FCS, Thermo Scientific), the prepared cells were mixed therein at 1×10⁶ cells/ml, and the resultant was then subjected to the reaction at 37°C for 3 hours. After the cells were washed two times in the AIM-V medium, the activity was measured using FACS CantoII. In order to elucidate the antigen degrading ability, DQ ovalbumin (Molecular Probes) was added at 10 µg/ml to the AIM-V medium containing 10% FCS, the reaction was carried out at 37°C for 30 minutes, and the reaction product was washed two times in the AIM-V medium, followed by the measurement using FACS CantoII. In the measurement of the antigen phagocytic activity and that of the antigen degrading ability, the cells subjected to the reaction at 4°C were used as negative controls, the values (ΔMFI) subtracted from the mean fluorescent intensity (MFI) obtained at 37°C were evaluated as the antigen phagocytic activity and the antigen degrading ability.

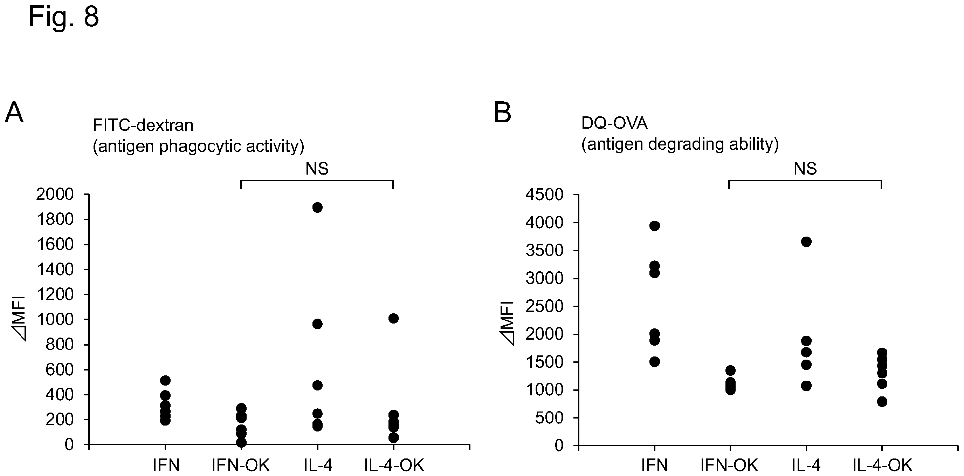

Fig. 8A shows the antigen phagocytic activity and Fig. 8B shows the antigen degrading ability represented by dot-plotting ΔMFI obtained by inspecting FITC-dextran uptake and the DQ ovalbumin (DQ-OVA) degrading ability with the use of the DCs prepared from monocytes obtained from cancer patients (n = 6). After the cells were stimulated with OK432, the antigen phagocytic activity and the antigen degrading ability of IFN DCs and IL-4 DCs lowered, and these DCs developed into mature DCs. No significant difference was observed between IFN-OK DCs and IL-4-OK DCs (NS = not significant). Significant difference was tested by the Wilcoxon signed rank test.

### 5. In vitro CTL induction

With the use of the DCs and the peripheral blood lymphocytes (PBL) prepared from a HLA-A^{∗}02:01 cancer patient (Patient ^{#}29), *in vitro* CTL induction was performed. Synthetic peptides MART-1 and 26-35 A27L (ELAGIGILTV) were adjusted to 20 µg/ml in the AIM medium, and the resultant was mixed with DCs. The resultant was pulsed at 37°C for 1 hour, treated with mitomycin C (MMC, 25 µg/ml; Kyowa Hakko Kogyo) for 1 hour, and washed two times in the AIM medium to prepare DCs. With the use of the AIM medium supplemented with IL-2 (Imunace, 2.5 U/ml; Shionogi), IL-7 (5 ng/ml; R&D Systems), and IL-15 (10 ng/ml; PeproTech), 1×10⁶ DCs were mixed with PBL at 1:10, and culture was conducted on a 12-well plate for 3 to 5 days. In accordance with the cell growth, the AIM-V medium containing 10% FCS was added, and culture was conducted for an additional 2 or 3 days to recover cells. The recovered cells were stained with CD8-FITC (Beckman Coulter), CD3-APC (eBioscience), and T-select HLA-A^{∗}02:01 MART-1 tetramer-ELAGIGILTV-PE (MBL), and MART-1-specific CTL was detected.

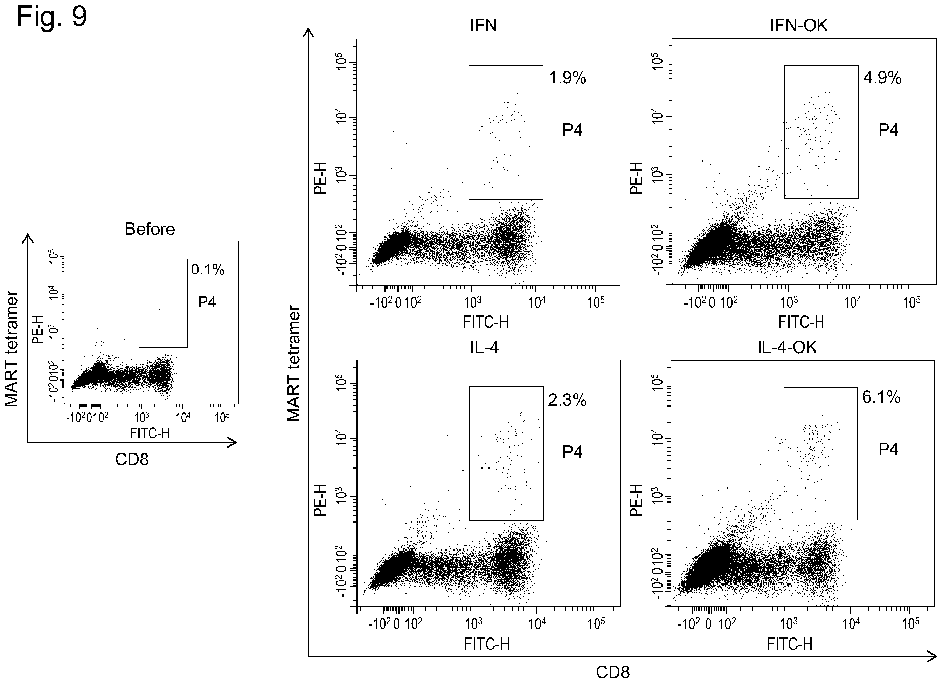

DCs were prepared from monocytes obtained from a HLA-A^{∗}02:01 cancer patient, the MART-1 antigens were pulsed, PBL was mixed with DCs at 10:1, and culture was conducted for 1 week. Thereafter, cytotoxic T lymphocytes (CTLs) were detected using the MART tetramer. The results are shown in Fig. 9. Numerical values shown in the panels indicates the proportion of MART tetramers⁺ in CD3⁺ cells and CD8⁺ cells induced by relevant DCs. The panel indicated with the term "Before" demonstrates the proportion of MART-specific CTLs in PBL before the initiation of induction. By stimulating IFN DCs or IL-4 DCs with OK432, the CTL-inducing ability was elevated, and no significant difference was observed therebetween (representative data (n = 3), significant difference is being tested while increasing the number n). The results demonstrate that stimulation with OK432 leads to the elevated antigen presenting activity and the antigen presenting activity of IFN-OK DC is equivalent to that of IL-4-OK DC.

### 6. Measurement of cytokine production

The density of the DCs was adjusted to 2 × 10⁵ cells/ml in the AIM-V medium, the resultant was inoculated into a 24-well plate, the culture supernatant was recovered 24 hours later, and the cytokine was then measured. With the use of interleukin-12 (IL-12 p70) and interferon-y (IFN-γ) (R&D Systems), a tumor necrosis factor-a (TNF-α), interleukin-6 (IL-6), interleukin-10, and the IL-10 ELISA kit (BD Bioscience), the reaction was allowed to proceed in accordance with the instructions, and measurement was then carried out using a spectrophotometer (MULTISKAN FC, Thermo Scientific).

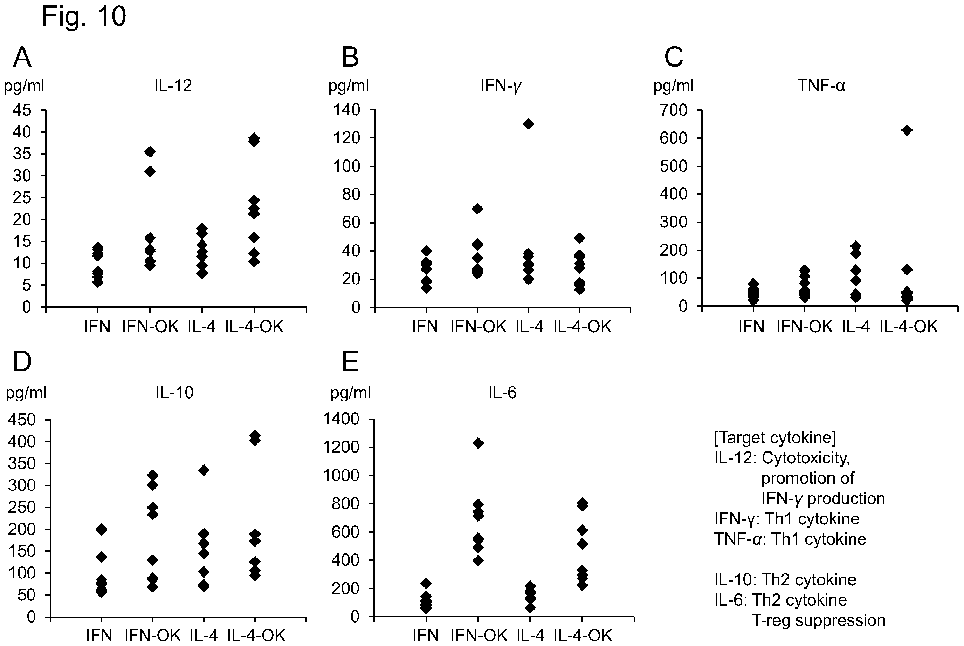

The results are shown in Fig. 10. In Fig. 10, A, B, C, D, and E each show IL-12-, IFN-γ-, TNF-α-, IL-10-, and IL-6-producing ability, respectively. After the addition of OK432, as shown in Fig. 10, the cytokine-producing ability tended to elevate, and no significant differences were observed in terms of cytokine production between IFN-OK DC and IL-4-OK DC. Significant differences were tested by the Wilcoxon signed rank test.

### 7. Cytotoxicity of the dendritic cells prepared

The cytotoxicity test was carried out in accordance with the method described in 1.

### (1) Cytotoxicity that attacks cancer cells

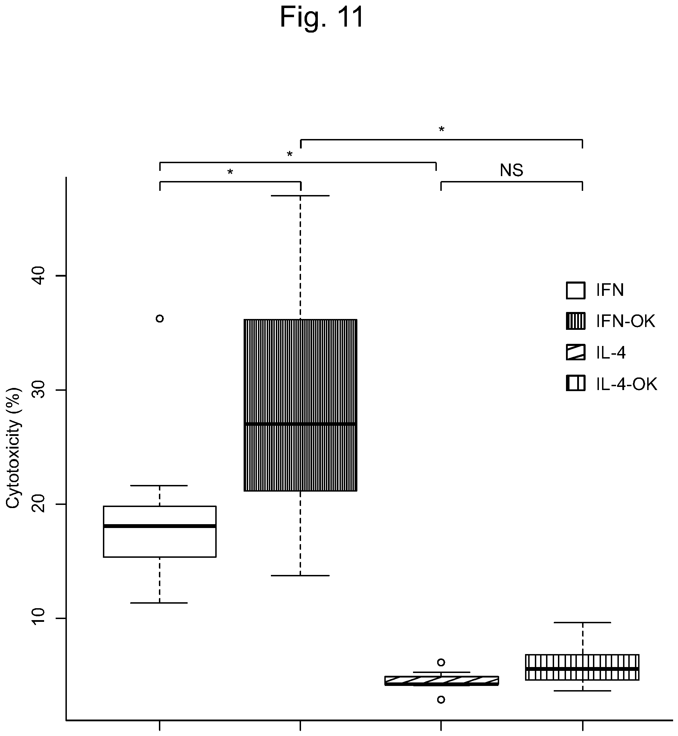

The DCs prepared from cancer patients and the chronic myeloid leukemia cells (K562) as the target cells were subjected to the reaction at E:T of 50:1 for 18 hours, and cytotoxicity was then examined. The results are shown in Fig. 11. As already reported, IFN DC was found to exhibit cytotoxicity, and the experimentation system was found to sufficiently function. Interestingly, stimulation of IFN DC with OK432 (IFN-OK DC) was found to significantly elevate the cytotoxicity, and IFN-OK DC was found to exhibit cytotoxicity superior to that of IL-4-OK DC to a significant extent. Significant difference was tested by the Wilcoxon signed rank test with the application of Bonfferoni correction (n = 8, NS = not significant, the symbol "*" indicates p < 0.05).

### (2) Correlation between CD56 expression and cytotoxicity

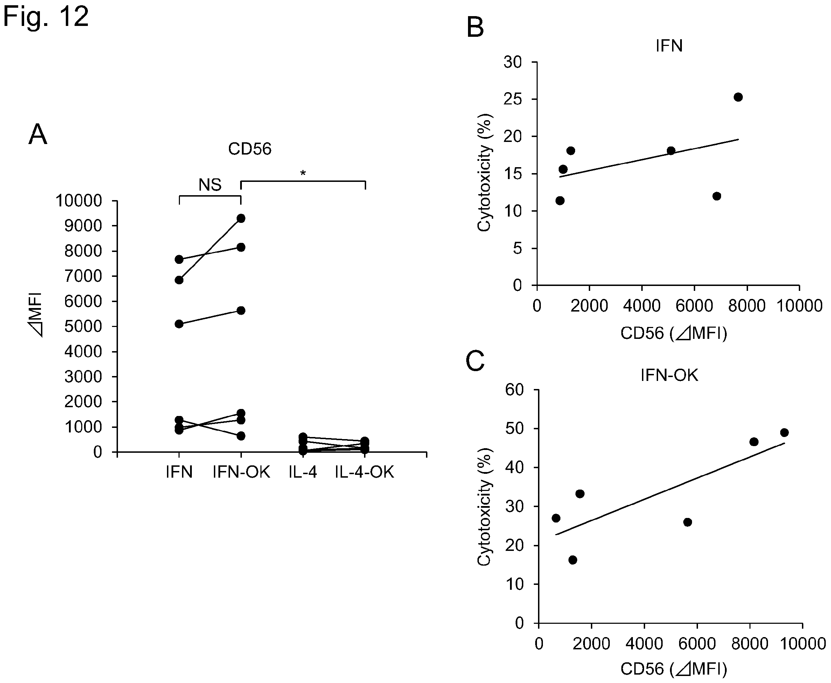

The prepared DCs were used to inspect the CD56 expression via FACS. K562 was designated as the target, IFN DC and IFN-OK DC were designated as the effectors, these cells were subjected to the reaction at E:T of 50:1 for 18 hours, and measurement was then carried out. Significant difference of IFN DC and IFN-OK DC was tested by the Wilcoxon signed rank test (NS: not significant; n = 6). The correlation between cytotoxicity and CD56 expression (ΔMFI) was evaluated using the Spearman's rank correlation coefficient, and significant differences were tested. The results are shown in Fig. 12. Fig. 12A demonstrates CD56 expression (ΔMFI) of the dendritic cells prepared by various methods, Fig. 12B demonstrates the correlation between CD56 expression and cytotoxicity in IFN DC, and Fig. 12C demonstrates the correlation between CD56 expression and cytotoxicity in IFN-OK DC. Regarding IFN DC, R is 0.637, p is 0.173, and n is 6. Regarding IFN-OK DC, R is 0.714, p is 0.136, and n is 6. Thus, the cytotoxicity of IFN-OK DC was found to be highly correlated with CD56 expression.

### (3) Mechanism of cell damage by IFN-OK DC

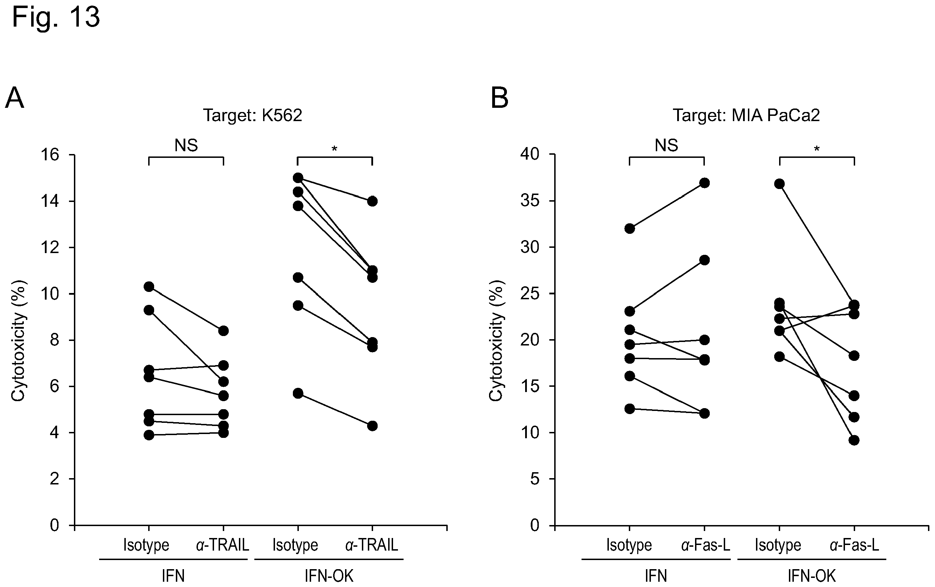

IFN DCs and IFN-OK DCs were suspended in the AIM medium containing 10% FCS, TRAIL-sensitive K562 cells were mixed with FasL (Fas ligand)-sensitive MIA PaCa2 at E:T of 50:1 one hour after the antibodies were added, and the cytotoxicity was examined via FACS 18 hours later. The results are shown in Fig. 13. Fig. 13A demonstrates the results concerning the K562 cells and Fig. 13B demonstrates the results concerning MIA PaCa2. Through treatment with the anti-TRAIL antibody and the anti-FasL antibody, the cytotoxicity on the K562 cells and that on MIA PaCa2 were found to be suppressed to a significant extent (n = 7, significant differences were tested by the Wilcoxon signed rank test, the symbol "*" indicates p < 0.05). The results demonstrate the involvement of TRAIL and FasL with the mechanism of cell damage by IFN-OK DC.

While IFN DC was reported to exert cytotoxicity on K562 with the aid of TRAIL, such cytotoxicity was not observed in this example. The influence of TRAIL and FasL imposed on IFN-OK DC stimulated with OK432 was reported for the first time.

In this example, statistical analysis was carried out using the statistic software R, significant differences were tested by the Wilcoxon signed rank test and the Tukey-Kramer test, and the correlational efficient and the significant differences were examined using Spearman's rank-order correlation.

### Example 2: Preparation of dendritic cells using IFN and OK432 (Part 2)

### 1. Preparation of dendritic cells

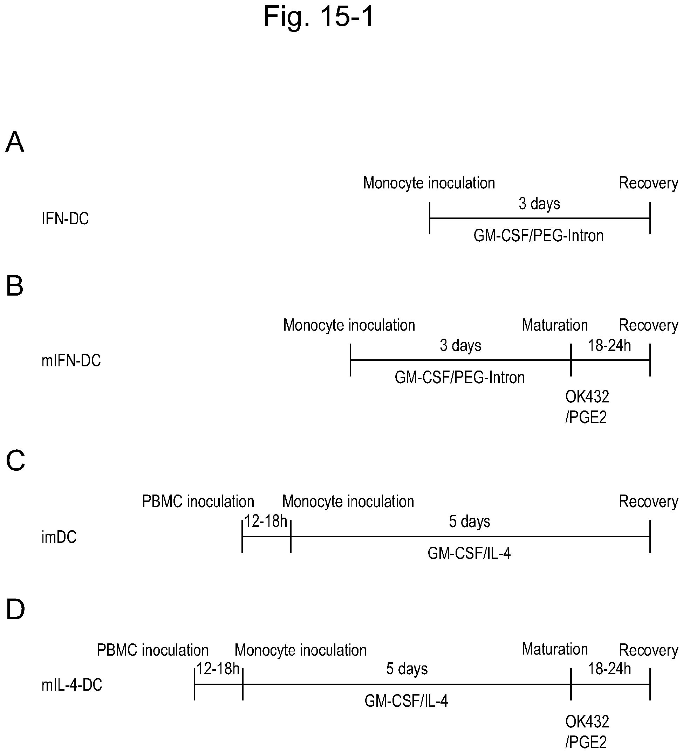

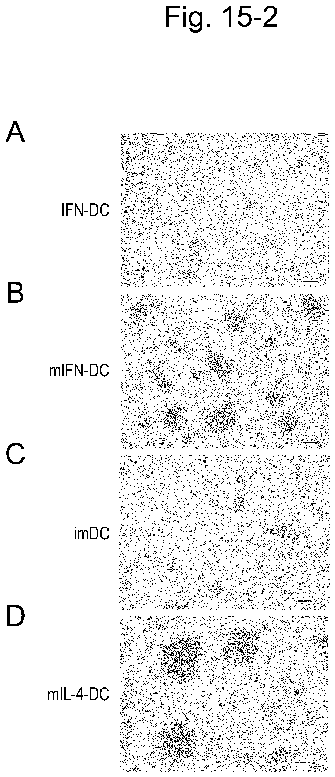

The CD14-positive cells sorted from the PBMCs were suspended in a medium containing GM-CSF (1,000 U/ml; Miltenyi Biotec) and a pegylated interferon α-2b formulation (PEGINTRON®, 1 µg/ml; MSD) at 2 to 4 × 10⁶ cells/ml, and culture was conducted in a non-adhesive culture dish for 3 days to prepare IFN-DCs (Fig. 15-1A). Culture was conducted in a maturation medium containing 10 µg/ml of OK432 (streptococcal preparation, Chugai Pharmaceutical Co, Ltd, Tokyo, Japan) and 50 ng/ml of PGE2 (Daiichi Fine Chemical Co, Ltd, Toyama, Japan), in addition to GM-CSF (1,000 U/ml; Miltenyi Biotec) for 1 day, and mIFN-DCs were then recovered (Fig. 15-1B). Separately, PBMCs were inoculated into an adhesive culture dish, non-adherent cells were removed, culture was conducted in a medium supplemented with GM-CSF (50 ng/ml; Gentaur) and IL-4 (50 ng/ml; R&D Systems) for 5 days to prepare imDCs (Fig. 15-1C), maturation was further carried out in a medium supplemented with OK432 (10 µg/ml) and PGE2 (50 ng/ml) for 18-24 hours, and mIL-4-DCs were then recovered (Fig. 15-1D). The DCs were observed under the phase contrast microscope at the time of recovery. The results are shown in Fig. 15-2A to Fig. 15-2D. A black scale bar represents 50 mm. As shown in Fig. 15-2A to Fig. 15-2D, in IFN-DCs dispersed in a culture dish (Fig. 15-2A), cell aggregation (cluster formation) was observed upon stimulation with OK432 (mIFN-DC, Fig. 15-2B), and a typical phenotype of mature DCs was observed. Cluster formation of mIFN-DC (Fig. 15-2A) tended to be similar to that of mIL-4-DC (Fig. 15-2D), and a phenotype of mature DCs was observed.

### 2. Enhanced cytotoxicity of IFN-DC by stimulation with OK432

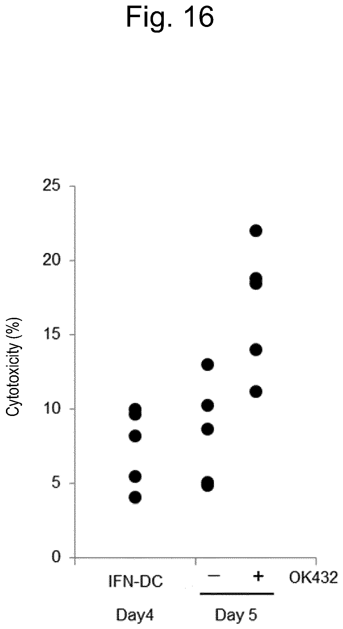

The culture solution of IFN-DC (supplemented with PEGINTRON) shown in Fig. 1 was supplemented with a medium supplemented with a cytokine cocktail prepared with or without the addition of OK432 (?/+) 4 days after the initiation of culture (Day 4), the cells were recovered on Day 5, and cytotoxic activity was assayed. The results are shown in Fig. 16. As shown in Fig. 16, the cytotoxicity (%) was significantly enhanced with the addition of OK432.

### 3. Induction of cell damage by OK432

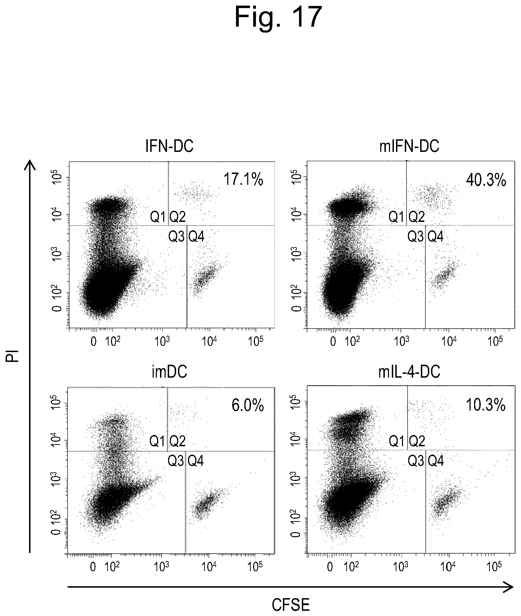

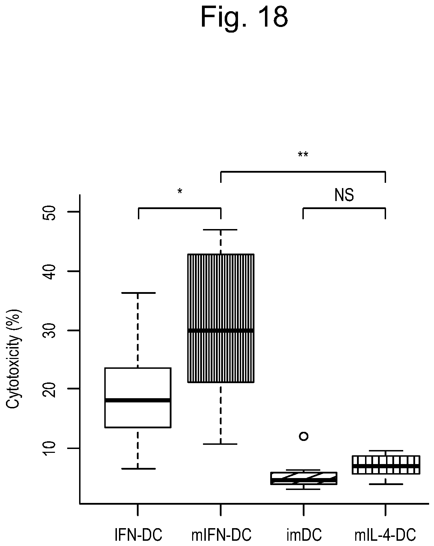

DCs were used as the effector cells (E), CFSE-stained K562 cells were used as the target cells (T), they were mixed at E:T of 50 : 1, the cells were stained with PI 18 hours later, and cell damage caused by DCs was examined (representative data (N = 11)). The results are shown in Figs. 17 and 18. Notations concerning DCs used in Fig. 17 correspond to those used in Fig. 15-1. Numerical values in the charts represent the percentage of the damaged K562 cells, and such values were determined on the basis of the percentage of PI-positive cells among the CFSE-stained K562 cells. As shown in Fig. 17, the highest cytotoxicity was observed in mIFN-DC prepared with the use of PEGINTRON and OK-432. Fig. 18 shows the cell damage imposed on K562 in the form of a box plot (N = 11). (*p < 0.05, **p < 0.01, NS: not significant).

### 4. Examination of cytotoxicity of mIFN-DC

### (1) Correlation between cytotoxicity and CD56 of mIFN-DC

CD56 expression of the prepared DCs was inspected via FACS.

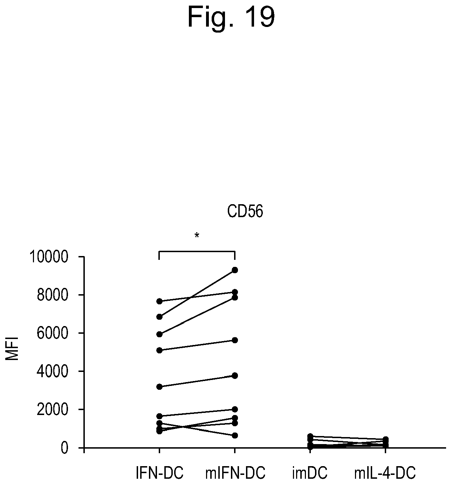

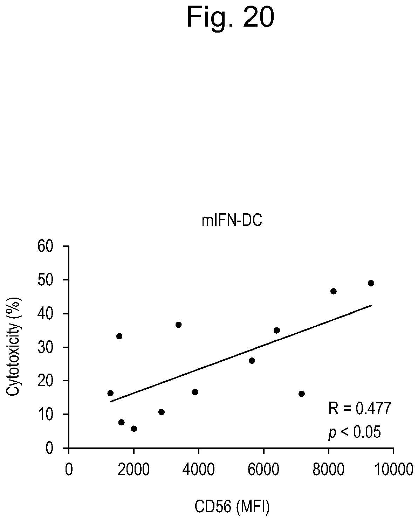

The mean fluorescence density (MFI) of CD56 of DCs is shown in the form of a dot plot and compared on the basis of maturation with OK432. The results are shown in Fig. 19 (*, p < 0.05). Notations concerning DCs used in Fig. 19 correspond to those used in Fig. 15-1. Fig. 20 shows the correlation between MFI and cytotoxicity of CD56 in mIFN-DC (N = 12). As shown in Figs. 19 and 20, the cytotoxicity of the mIFN-DC prepared with the use of PEGINTRON and OK-432 was correlated with the CD56 expression level.

### (2) Involvement of cell contact with induction of cell damage caused by OK432

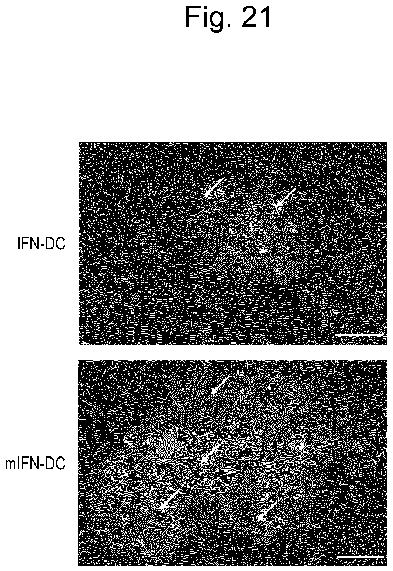

The CFSE-labeled K562 cells were mixed with the PKH26-stained IFN-DCs or IFN-DCs at 1:1, the cells were subjected to the reaction for 18 hours, and the cells were further subjected to the reaction with DAPI (blue) for 5 minutes. Thereafter, the cells were observed under a fluorescence microscope. Fig. 21 shows microscopic images. White arrows indicate DAPI-positive dead cells and a white scale bar indicates 50 µm. As shown in Fig. 21, clusters formed by IFN-DCs and the K562 cells were small (the upper image, Fig. 21), cells were dispersed, and the number of dead cells was very small. In contrast, clusters formed by the mIFN-DCs and the K562 cells were large and apparent (the lower image, Fig. 21), and many dead cells were observed in the K562 cells within the cluster. The results suggest that cluster formation; that is, intercellular contact, is critical for induction of cell damage.

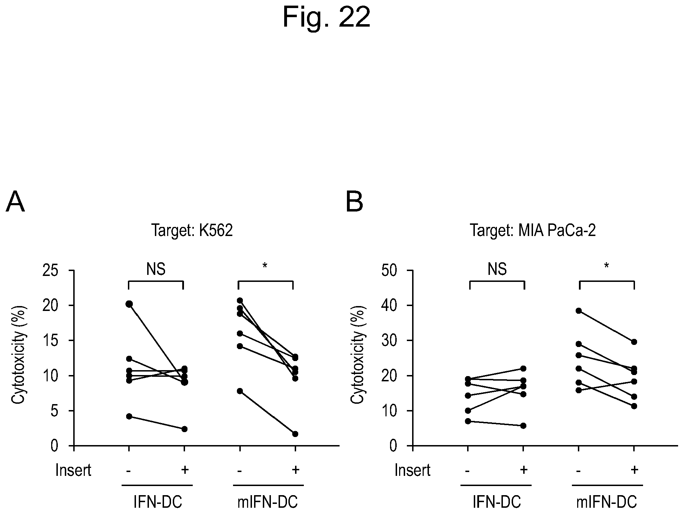

Subsequently, K562 or MIA PaCa-2 was used as the target, and cytotoxicity caused by IFN-DC or mIFN-DC was inspected based on the presence or absence of transwell membranes (0.4 mm) (N = 6). The results are shown in Fig. 22. As shown in Fig. 22, when the K562 cells were the target cells (Fig. 22A), no difference was observed in cell damage caused by IFN-DC in terms of the presence or absence of transwell membranes (Inserts) inhibiting cell contact. In contrast, cell damage caused by mIFN-DC was found to decrease to a significant extent when cell contact was inhibited (Insert+). That is, cell contact may be critical for enhanced cell damage caused by OK432-stimulated mIFN-DC. Since such phenomenon was not limited to the K562 cells but was also observed in MIA PaCa-2 (Fig. 22B), such phenomenon was considered to be a common mechanism of cell damage.

### 5. Induction of cell damage mediated by TRAIL and Fas ligand by OK432

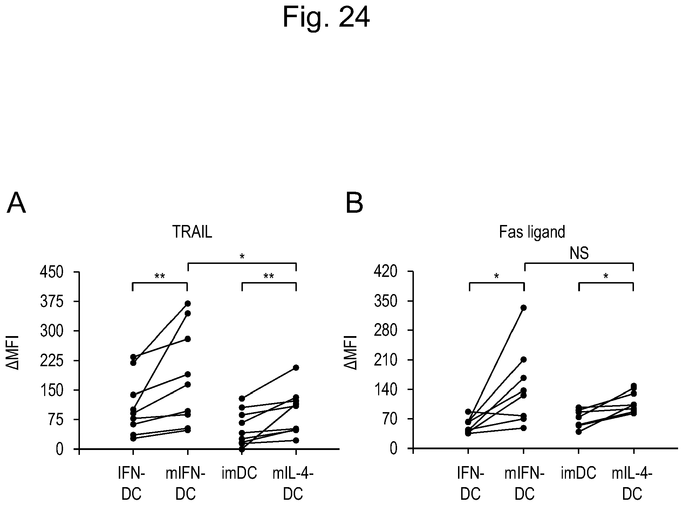

Subsequently, ΔMFI of TRAIL (N = 9) and that of the Fas ligand (N = 8) on the DC cell surface were compared (i.e., MFI determined by subtracting an isotype control from the relevant specific antibody). The results are shown in Fig. 24. As shown in Fig. 24, variations in expression were precisely evaluated on the basis of MFI, and statistically significant differences were examined. As a result, stimulation with OK432 was found to significantly increase TRAIL and Fas ligand expression levels of mIFN-DC and mIL-4-DC. Compared with mIL-4-DC, the TRAIL expression level in mIFN-DC was significantly higher. Accordingly, a high TRAIL expression level in IFN-DC was considered to be involved with such phenomenon. It was thus suggested that inhibition of TRAIL would inhibit cell damage and a high TRAIL expression level would affect cell damage in mIFN-DC.

### 6. Comparison of phenotypes of DCs matured by OK432

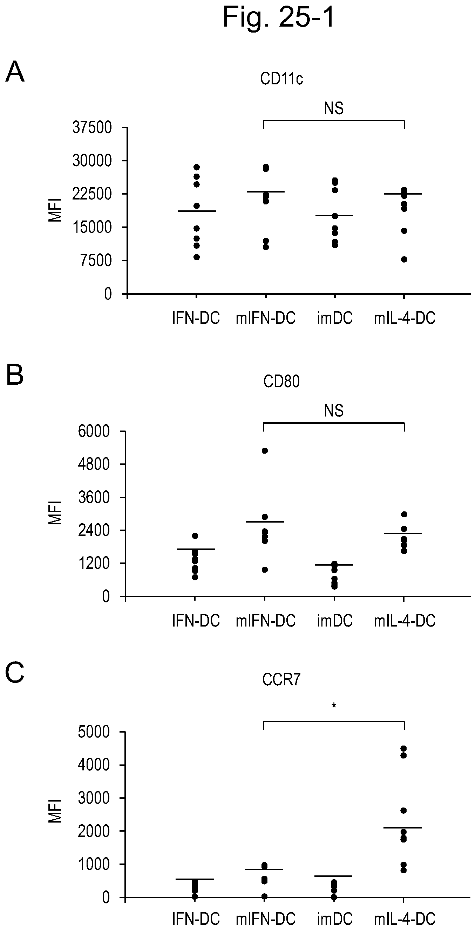

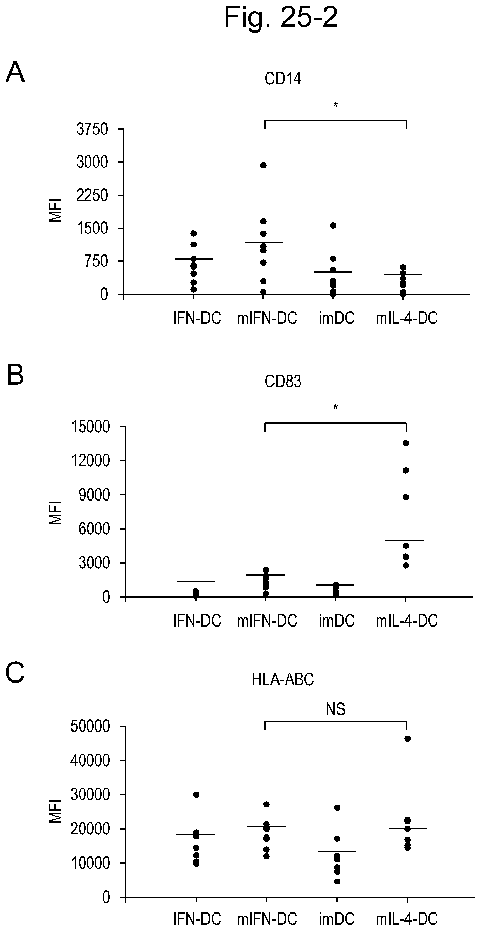

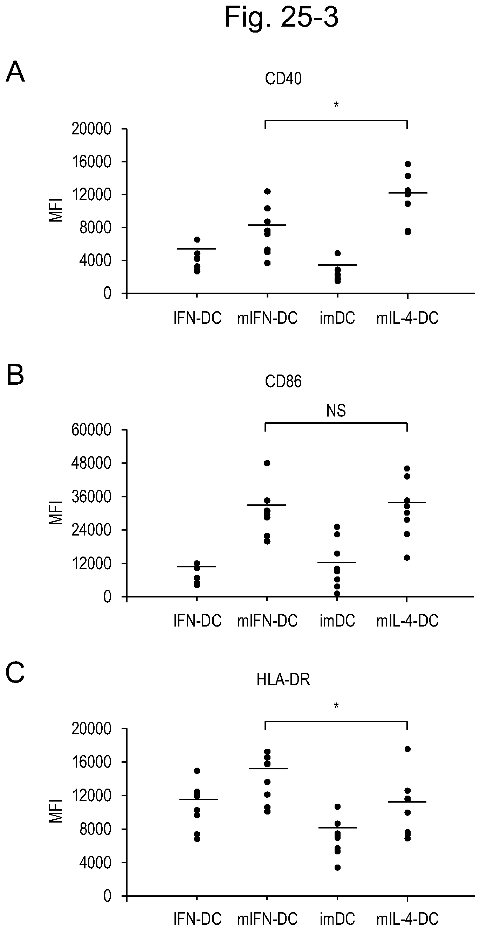

DCs were prepared from PBMCs obtained from cancer patients (N = 8), typical DC markers were analyzed via flow cytometry, and the mean fluorescence intensity (MFI) was measured. MFIs are shown in the form of a dot plot in Fig. 25-1 to Fig. 25-3. Fig. 25-1 shows values concerning CDllc (Fig. 25-1A), CD80 (Fig. 25-1B), and CCR7 (Fig. 25-1C), Fig. 25-2 shows values concerning CD14 (Fig. 25-2A), CD83 (Fig. 25-2B), and HLA-ABC (Fig. 25-2C), and Fig. 25-3 shows values concerning CD40 (Fig. 25-3A), CD86 (Fig. 25-3B), and HLA-DR (Fig. 25-3C).

### 7. Functions of mature DCs prepared by OK432

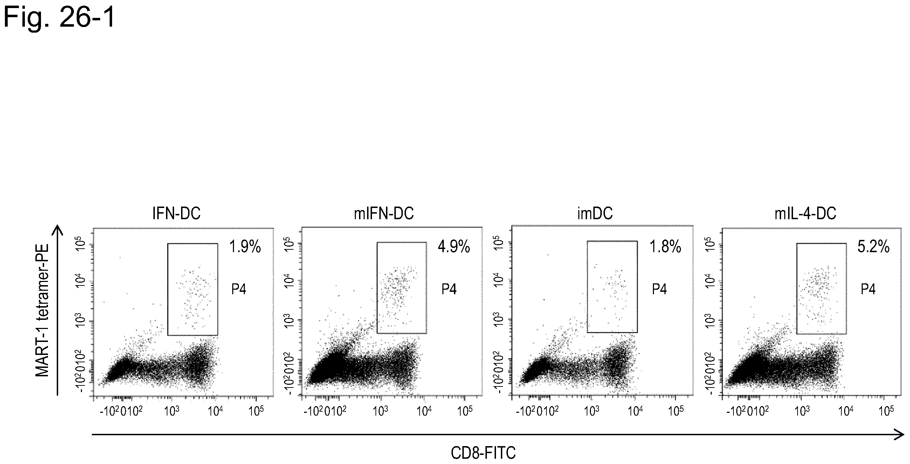

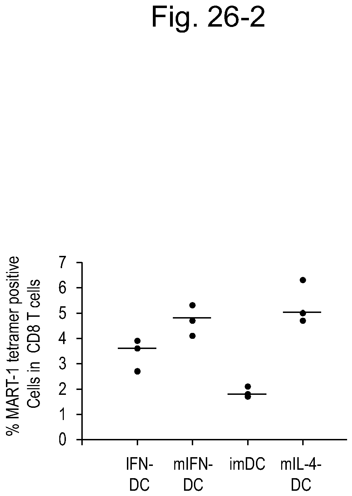

DCs pulsed with Mart antigens (i.e., cancer antigens specific to human melanoma cells) were mixed with PBL, culture was conducted for 1 week, CD3-positive lymphocytes were subjected to gating, and the results were plotted as CD8-FITC along the horizontal axis and as MART-1 tetramer-PE. Thus, Mart-1-specific CTL induction was inspected. Numerical values in the panels represent the percentage of MART-specific CTL among CD3- and CD8-positive cells. The results are shown in Fig. 26-1 and Fig. 26-2 (a dot plot). As shown in Fig. 26-1 and Fig. 26-2, no differences were observed between mIFN-DC and mIL-4-DC in respect of the antigen presenting activity.

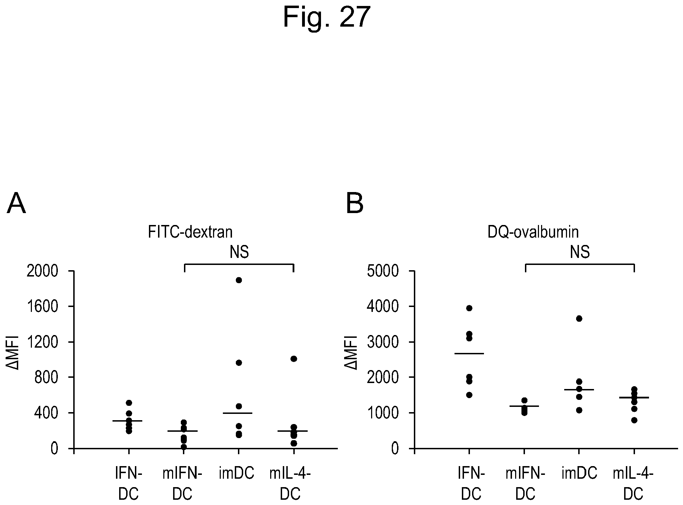

The antigen phagocytic activity was inspected based on FITC-dextran uptake and the antigen degrading ability was confirmed based on the fluorescence intensity of DQ-Ovalbumin. The value obtained for the negative control subjected to the reaction at 4°C was subtracted from the value obtained for the sample subjected to the reaction at 37°C to determine ΔMFI, and the obtained ΔMFI is shown in the form of a dot plot (n = 6). The results are shown in Fig. 27. As shown in Fig. 27, compared with IFN-DC and imDC, the amount of FITC-dextran uptake into OK432-stimulated mIFN-DC and mIL-4-DC decreased to a significant extent, and similar activity was observed (Fig. 27A). Specifically, the antigen phagocytic activity of mIFN-DC was similar to that of mIL4-DC. As a result of fluorescence intensity measurement based on DQ-ovalbumin degradation, the antigen degrading ability was found to significantly decrease upon stimulation with OK432, and the antigen degrading ability of mIFN-DC was found to be similar to that of mIL-4-DC (Fig. 27B). Thus, mIFN-DC and mIL-4-DC were found to exhibit similar antigen phagocytic activity and antigen presenting activity of mature DCs.

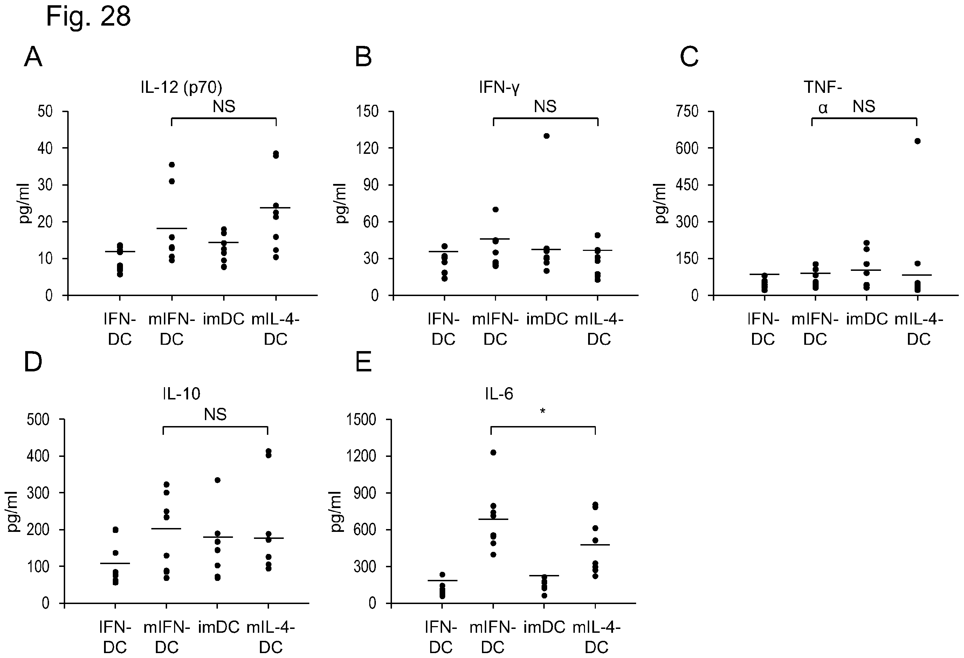

DCs (2 × 10⁵ cells/ml) were inoculated on a 24-well plate, culture was conducted in the AIM medium for 24 hours, the resulting supernatant was used to inspect production of cytokines (IL-12, IFN-γ, TNF-α, IL-10, and IL-6) via ELISA (N = 8). The results are shown in Fig. 28. As shown in Fig. 28, the cytokine IL-12 (p70) and IFN-γ that would enhance CTL induction elevated upon stimulation with OK432 were also observed in mIFN-DC as well as in mIL-4-DC. No variation was observed in TNF-α, which is a soluble tumor necrosis factor. The production level for IL-10 that would inhibit CTL induction was similar between mIFN-DC and mIL-4-DC. The production level for IL-6 that would exert inhibitory functions on regulatory T cells that would lower the CTL inducing capacity was significantly higher in mIFN-DC than in mIL-4-DC, compared with mIl-4-DC. The results demonstrate that, except for IL-6 production, mIFN-DC has the similar cytokine producing ability of mature DC as with mIL-4DC.

### Industrial Applicability

The dendritic cells prepared by the method of the present invention can be used for dendritic cell therapy.

## Claims

1. A method for preparing cytotoxic dendritic cells from monocytes comprising subjecting monocytes which were isolated from the peripheral blood to non-adhesive culture in the presence of GM-CSF and pegylated interferon α and further conducting non-adhesive culture with the addition of prostaglandin E2 and OK432.

2. The method for preparing dendritic cells from monocytes according to claim 1, wherein, following the non-adhesive culture conducted for 2 to 5 days in the presence of GM-CSF and pegylated interferon α, non-adhesive culture is conducted for an additional 1 or 2 days with the addition of prostaglandin E2 and OK432.

3. The method for preparing dendritic cells from monocytes according to claim 1 or 2, wherein the monocytes are cultured with the use of 100 U/ml to 10,000 U/ml of GM-CSF, 500 ng/ml to 5 µg/ml of pegylated interferon α, 5 ng/ml to 50 ng/ml of prostaglandin E2, and 5 µg/ml to 50 µg/ml of OK432.

4. The method for preparing dendritic cells from monocytes according to any one of claims 1 to 3, wherein the percentage of viable dendritic cells is 90% or higher and the yield indicating the proportion of the number of the obtained dendritic cells relative to the number of monocytes at the time of culture is 15% or higher.

5. The method for preparing dendritic cells from monocytes according to any one of claims 1 to 4, wherein the obtained dendritic cells are positive for CD11c, CD14, CD40, CD56, CD80, CD86, HLA-ABC, and HLA-DR and negative for CD83 and CD197.

6. The method for preparing dendritic cells from monocytes according to any one of claims 1 to 5, wherein, when effector cells (DCs) and target cells (the chronic myeloid leukemia cell line K562) are subjected to culture at a ratio of 50:1 for 18 hours, the cytotoxicity of the dendritic cells measured as the proportion of the target cells as dead cells is 30% or higher.

7. An agent for inducing and differentiating cytotoxic dendritic cell (DC) from monocytes comprising GM-CSF, pegylated interferon α, prostaglandin E2, and OK432.

8. The agent for inducing cytotoxic dendritic cell differentiation from monocytes according to claim 7, which comprises an agent for inducing and differentiating immature dendritic cell comprising GM-CSF and pegylated interferon α and an agent for dendritic cell maturation comprising prostaglandin E2 and OK432.

## Patentansprüche

1. Verfahren zum Herstellen zytotoxischer dendritischer Zellen aus Monozyten, umfassend ein Aussetzen von Monozyten, die aus dem peripheren Blut isoliert wurden, an eine nicht-adhäsive Kultur in Anwesenheit von GM-CSF und pegyliertem Interferon α und ferner Durchführen einer nicht-adhäsiven Kultur mit dem Hinzufügen von Prostaglandin E2 und OK432.

2. Verfahren zum Herstellen von dendritischen Zellen aus Monozyten nach Anspruch 1, wobei im Anschluss an die nicht-adhäsive Kultur, die über 2 bis 5 Tage in Anwesenheit von GM CSF und pegyliertem Interferon α durchgeführt wird, eine nicht-adhäsive Kultur über weitere 1 oder 2 Tage mit dem Hinzufügen von Prostaglandin E2 und OK432 durchgeführt wird.

3. Verfahren zum Herstellen von dendritischen Zellen aus Monozyten nach Anspruch 1 oder 2, wobei die Monozyten unter Verwendung von 100 U/ml bis 10.000 U/ml GM-CSF, 500 ng/ml bis 5 µg/ml pegyliertem Interferon α, 5 ng/ml bis 50 ng/ml Prostaglandin E2 und 5 µg/ml bis 50 µg/ml OK432 kultiviert werden.

4. Verfahren zum Herstellen von dendritischen Zellen aus Monozyten nach einem der Ansprüche 1 bis 3, wobei der Prozentsatz von lebensfähigen dendritischen Zellen 90 % oder höher ist und der Ertrag, der den Anteil der Anzahl von erlangten dendritischen Zellen in Bezug auf die Anzahl der Monozyten zum Zeitpunkt der Kultur angibt, 15 % oder höher ist.

5. Verfahren zum Herstellen von dendritischen Zellen aus Monozyten nach einem der Ansprüche 1 bis 4, wobei die erlangten dendritischen Zellen positiv für CD11c, CD14, CD40, CD56, CD80, CD86, HLA-ABC und HLA-DR und negativ für CD83 und CD197 sind.

6. Verfahren zum Herstellen von dendritischen Zellen aus Monozyten nach einem der Ansprüche 1 bis 5, wobei, wenn Effektorzellen (DCs) und Zielzellen (die Zelllinie K562 für chronisch-myeloische Leukämie) 18 Stunden einer Kultur in einem Verhältnis von 50:1 ausgesetzt werden, die Zytotoxizität der dendritischen Zellen, gemessen als Anteil der Zielzellen als tote Zellen, 30 % oder höher ist.

7. Mittel zum Induzieren und Differenzieren zytotoxischer dendritischer Zellen (DC) aus Monozyten, umfassend GM-CSF, pegyliertes Interferon α, Prostaglandin E2 und OK432.

8. Mittel zum Induzieren und Differenzieren aus Monozyten nach Anspruch 7, das ein Mittel zum Induzieren und Differenzieren unreifer dendritischer Zellen, umfassend GM-CSF und pegyliertes Interferon α, und ein Mittel zur Reifung dendritischer Zellen, umfassend Prostaglandin E2 und OK432, umfasst.

## Revendications

1. Procédé de préparation de cellules dendritiques cytotoxiques à partir de monocytes comprenant la soumission de monocytes qui ont été isolés du sang périphérique à une culture non adhésive en présence de GM-CSF et d'interféron α pégylé et réalisant en outre une culture non adhésive avec l'ajout de prostaglandine E2 et d'OK432.

2. Procédé de préparation de cellules dendritiques à partir de monocytes selon la revendication 1, dans lequel, suite à la culture non adhésive réalisée pendant 2 à 5 jours en présence de GM-CSF et d'interféron α pégylé, une culture non adhésive est réalisée pour une durée supplémentaire de 1 ou 2 jours avec l'ajout de prostaglandine E2 et d'OK432.

3. Procédé de préparation de cellules dendritiques à partir de monocytes selon la revendication 1 ou 2, dans lequel les monocytes sont cultivés en utilisant 100 U/ml à 10 000 U/ml de GM-CSF, 500 ng/ml à 5 µg/ml d'interféron α pégylé, 5 ng/ml à 50 ng/ml de prostaglandine E2, et 5 µg/ml à 50 µg/ml d'OK432.

4. Procédé de préparation de cellules dendritiques à partir de monocytes selon l'une quelconque des revendications 1 à 3, dans lequel le pourcentage de cellules dendritiques viables est de 90% ou plus et le rendement indiquant la proportion du nombre de cellules dendritiques obtenues par rapport au nombre des monocytes au moment de la culture est de 15% ou plus.

5. Procédé de préparation de cellules dendritiques à partir de monocytes selon l'une quelconque des revendications 1 à 4, dans lequel les cellules dendritiques obtenues sont positives pour CD11c, CD14, CD40, CD56, CD80, CD86, HLA-ABC et HLA-DR et négatives pour CD83 et CD197.

6. Procédé de préparation de cellules dendritiques à partir de monocytes selon l'une quelconque des revendications 1 à 5, dans lequel, lorsque des cellules effectrices (DC) et des cellules cibles (la lignée cellulaire de leucémie myéloïde chronique K562) sont soumises à une culture dans un rapport de 50 à 1 pendant 18 heures, la cytotoxicité des cellules dendritiques mesurée comme la proportion des cellules cibles en tant que cellules mortes est de 30% ou plus.

7. Agent pour induire et différencier des cellules dendritiques cytotoxiques (DC) à partir de monocytes comprenant du GM-CSF, de l'interféron α pégylé, de la prostaglandine E2 et de l'OK432.

8. Agent pour induire une différenciation des cellules dendritiques cytotoxiques à partir de monocytes selon la revendication 7, qui comprend un agent pour induire et différencier des cellules dendritiques immatures comprenant du GM-CSF et de l'interféron α pégylé et un agent pour la maturation des cellules dendritiques comprenant de la prostaglandine E2 et de l'OK432.
